# EUROPEAN PATENT APPLICATION

(11) **EP 4 596 569 A1**
(43) Date of publication of application: **06.08.2025**
(21) Application number: 24305198.4
(22) Date of filing: 05.02.2024
(51) Int. Cl.: C07K 1/113

(54) **SITE SPECIFIC ANTIBODY DRUG CONJUGATES AND THEIR PROCESS OF PREPARATION**

(71) Applicant: Syndivia, 67083 Strasbourg (FR)
(72) Inventor: DATTLER, Damien, 67000 Strasbourg (FR); SCHLIMPEN, Fabian, 67100 Strasbourg (FR); SEMENCHENKO, Constantin, 67400 Illkirch-Graffenstaden (FR); KONIEV, Oleksandr, 67200 Strasbourg (FR); KOLODYCH, Sergii, 67200 Strasbourg (FR)
(74) Representative: Lavoix

(57) **Abstract**

The present application is directed to novel site specific antibody-drug conjugates and the process leading to the same.

## Description

Modern cancer therapeutics aim at increasing specificity for cancer cells thus reducing toxicity for healthy tissues.

Antibody drug conjugates (ADCs) represent a promising class with this potential exploiting their antigen-specificity for cancer-related targets to deliver cytotoxic compounds to cancer cells.

ADCs are highly valuable tools in cancer treatment, as they enable the targeted delivery of potent cytotoxins to cancer cells. However, the current methods used to create ADCs still have various limitations, particularly in terms of site-selectivity during payload conjugation and controlling the drug-to-antibody ratio (DAR), i.e., the number of drugs per antibody. Considering the accelerated clearance and reduced safety profile witnessed in first generation ADCs featuring drug-to-antibody ratios (DARs) frequently reaching eight, a shift in the prevailing paradigm has occurred in favour of conjugates with lower DAR values. Particularly challenging is the generation of ADCs with site specificity for the conjugation and/or controlled degree of conjugation.

Recent endeavours have also pivoted towards disulfide re-bridging reagents specifically reacting with cysteine-thiols from (partially) reduced antibodies.

These re-bridging agents also furnish supplementary functional moieties to enable subsequent payload-linker conjugation.

Various re-bridging strategies are under investigation. They should ensure the covalent linkage of the heavy and light chains of the antibodies.

In mAbs vol.11 (3), 2019, White et al disclose a re-bridging strategy to prepare homogenous ADCs with DAR of one using an engineered antibody and a PBD dimer. However, the proposed strategy requires an engineered antibody and the use of a long linker, which can potentially impact the PK/PD parameters of the ADC.

WO 2020/164561 discloses a process for preparing antibody-drug conjugates with improved homogeneity. However, said process leads to mixtures mainly comprising ADC with a drug-to-antibody ratio of 4, in mixture with DAR of 0, 2 and 6.

There is thus still a need to provide more versatile methods, which may apply to both native and engineered antibodies, allowing efficient covalent linkage of cysteine residues with site specificity and controlled degree of conjugation.

The present invention is based *inter alia* on the provision of a versatile protein complex of coordination and a novel process for synthesizing the same. Said protein complex is a key intermediate that may conveniently lead to various drug conjugates having a site-specific conjugation.

### BRIEF DESCRIPTION

According to a first object, the present invention concerns a coordination complex of protein with a divalent metal atom M, represented by formula (II):

[Ab'-M] (II)

Wherein
Ab' is a protein or fragment thereof comprising two heavy chains and two light chains, each chain comprising one or more cysteine residue involved in inter chains disulfide bridges, wherein each heavy chain comprises one N-proximal inter(heavy chains) cysteine residue that is located nearest to the N-terminal end of said heavy chain,
And wherein the Sulfur atom of each said N-proximal inter(heavy chains) cysteine residue is reduced, and the remaining interchain cysteine residues form interchain disulfide bridges; M is a divalent transition metal;
Wherein M bridges together the two reduced Sulfur atoms, so as to form the following group:
   -S-M-S- within the coordination complex;
   And the salts thereof;
   Characterized in that the complexion of M is site specific.

The present invention distinctively relies on site-specificity for the coordination for e.g. monoclonal IgG antibodies.

This is based on targeting one of the two inter-heavy chain disulfide bonds in the hinge region, specifically the bond that is nearest to the N-terminal end of the Protein's heavy chains.

The invention involves the selective modification of this identified disulfide bond, distinguishing it from the second inter-heavy chain disulfide bond and the two inter-heavy-light chain disulfide bonds.

This targeted approach facilitates the creation of modified proteins, such as IgG molecules with improved specificity and potential therapeutic efficacy.

According to a further object, the present invention also concerns the process of preparation of the complex of formula (II) as defined herein, said process comprising: Reacting a protein complex of formula (I):

[Ab'_{Red}-M] (I)

With an oxidizing agent;
wherein
Ab'_{Red} is a protein or fragment thereof comprising two heavy chains and two light chains, each chain comprising one or more cysteine residue involved in inter chains disulfide bridges,
wherein each heavy chain comprises one N-proximal inter(heavy chains) cysteine residue that is located nearest to the N-terminal end of said heavy chain,
And wherein each Sulfur atom of each said N-proximal inter(heavy chains) cysteine residue is reduced and the two reduced Sulfur atoms form together with the M atom the following group: -S-M-S- within the coordination complex;
and the Sulfur atoms of the remaining interchain cysteine residues are reduced in the form of thiol -SH groups;
M is defined as in formula (II).

According to third object, the present invention also concerns a coordination complex of protein of formula (I):

[Ab'_{Red}-M] (I)

Where Ab'_{Red} and M are defined herein.

According to a fourth object, the invention also concerns the process of preparation of the complex of formula (I) as defined herein, said process comprising:
- reacting Ab with a reducing agent so as to form Ab_{Red}, followed by
- reacting Ab_{Red} with 0.5 to 2.5, preferably between 1 and 2 equivalents, more preferably between 1 and 1.5, still more preferably around 1.4 equivalent of a salt of metal M so as to form the Metal coordination complexes [Ab'_{Red}-M];

Wherein
Ab is a protein or fragment thereof comprising two heavy chains and two light chains, each chain comprising one or more cysteine residue involved in inter chains disulfide bridges,
Ab_{Red} refers to reduced Ab wherein all Sulfur atoms of said interchain cysteine residues are reduced and in the form of thiol -SH groups.

According to a fifth object, the invention also concerns a protein conjugate of formula (III):

(T)ₚ-Ab'-(L-(Payload)ₘ)ₙ (III)

Having
- a controlled drug-to-antibody ratio (DAR) of (m x n); and
- a site specific conjugation at one or two of the N-proximal inter(heavy chains) cysteine residue that is located nearest to the N-terminal end of said heavy chain;

Where in formula (III):
Ab' is a protein or fragment thereof comprising two heavy chains and two light chains, each chain comprising one or more cysteine residue involved in inter chains disulfide bridges,
wherein each heavy chain comprises one N-proximal inter(heavy chains) cysteine residue that is located nearest to the N-terminal end of said heavy chain,
And wherein each Sulfur atom of each said N-proximal inter(heavy chains) cysteine residue is reduced and covalently bound to either -(L-(Payload)ₘ or optional T fragment, and the remaining interchain cysteine residues form interchain disulfide bridges;
n is 1 or 2;
m is 1, 2, 3, 4, 5 or 6;
p is 0 or 1;
T, if present, is a terminal group covalently bound to one of said two reduced Sulfur atoms of said N-proximal inter(heavy chains) cysteine residues of Ab' that is not covalently bound to L;
each L, identical or different, is a linear or branched di-, tri-, tetra-, penta-, hexa- or hepta-valent linker moiety covalently binding Payload to said Ab', where said L is attached to Ab' through one or two of said reduced Sulfur atoms of said N-proximal inter(heavy chains) cysteine residue and binds said Ab' with one or more Payload;
Payload is a molecule exerting a biological activity selected from the group consisting in diagnostic agents, therapeutic agents and labelling agents.

According to a sixth object, the invention also concerns a process of preparation of a protein conjugate of formula (III) as defined herein, said process comprising complexing said metal atom from said compound of formula (II) as defined herein and conjugating said de-complexed compound with 1 to n conjugating fragments L-(Payload)ₘ as defined herein.

According to a seventh object, the invention also concerns the therapeutical use of the compounds of formula (III).

### DETAILED DESCRIPTION

### The protein

Typically, said Protein may be a naturally occurring protein or a synthetic protein, preferably a naturally occurring antibody or a synthetic antibody, such as an engineered antibody, herein referred to as Protein.

Said Protein typically comprises one or more polypeptide chains made of amino acids, it being provided that the cysteine residues may form covalent bonds through the formation of disulphide bridges, typically between different peptide chains within Protein. These are called herein « interchain cysteine residues ».

Typically, said naturally occurring or synthetic protein comprises two heavy chains and two light chains, where preferably each heavy chain comprises three cysteine residues and each light chain comprises one cysteine residue in the hinge region.

These chains are covalently bridged together by disulfide bonds created between the sulfur atom of the cysteine residues.

As used herein, the hinge region refers to an amino acid stretch in the central part of the two heavy chains, which links these two chains by disulfide bonds, whose respective sulfur atoms belong to cysteine residues.

Typically, the heavy chains are bridged together by the two interchain disulfide bridges created between two pairs of cysteine residues, and each heavy chain is further bridged to a light chain by a further disulfide bridge created between two cysteine residues.

More specifically, said Protein or fragment thereof comprises two heavy chains and two light chains, each chain comprising one or more cysteine residue involved in inter chains disulfide bridges, and wherein each heavy chain comprises one N-proximal inter(heavy chains) cysteine residue that is located nearest to the N-terminal end of said heavy chain. These two N-proximal inter(heavy chains) cysteine residues (also called herein "N-proximal cysteines") are a key site for site-specific conjugation according to the invention.

Without being bound by theory, it is hypothesized that these N-proximal cysteines are adjacent to or close to histidine residues that may confer the appropriate reactivity for complex formation with a metal atom, that will then lead to a reactive position for conjugation.

Said N-proximal cysteines are often referred to as Cysteine-239.

Protein may preferably be an antibody, in particular Immunoglobulin G, or a derivative thereof, such as IgG1 or IgG4 isotype, for example, IgG1 isotype.

According to an embodiment, said synthetic or naturally occurring protein comprises one or more aminoacid, such as peptide or a protein.

Typically, said Protein is a monoclonal antibody, such as human, humanized, mouse or chimeric antibodies. Typical antibodies include trastuzumab, bevacizumab, cetuximab, panitumumab, ipilimumab, rituximab, alemtuzumab, ofatumumab, gemtuzumab, brentuximab, ibritumomab, tositumomab, pertuzumab, adecatumumab, IGN101, INA01 labetuzumab, hua33, pemtumomab, oregovomab, minretumomab (CC49), cG250, J591, MOv-18, farletuzumab (MORAb-003), 3F8, ch14,18, KW-2871, hu3S193, IgN311, IM-2C6, CDP-791, etaracizumab, volociximab, nimotuzumab, MM-121, AMG 102, METMAB, SCH 900105, AVE1642, IMC-A12, MK-0646, R1507, CP 751871, KB004, III A4, mapatumumab, HGS-ETR2, CS-1008, denosumab, sibrotuzumab, F19, 81C6, pinatuzumab, lifastuzumab, glembatumumab, coltuximab, lorvotuzumab, indatuximab, anti-PSMA, MLN-0264, ABT-414, milatuzumab, ramucirumab, abagovomab, abituzumab, adecatumumab, afutuzumab, altumomab pentetate, amatuximab, anatumomab, anetumab, apolizumab, arcitumomab, ascrinvacumab, atezolizumab, bavituximab, bectumomab, belimumab, bivatuzumab, brontictuzumab, cantuzumab, capromab, catumaxomab, citatuzumab, cixutumumab, clivatuzumab, codrituzumab, conatumumab, dacetuzumab, dallotuzumab, daratumumab, demcizumab, denintuzumab, depatuxizumab, derlotuximab, detumomab, dinutuximab, drozitumab, duligotumab, durvalumab, dusigitumab, ecromeximab, edrecolomab, elgemtumab, emactuzumab, enavatuzumab emibetuzumab, enfortumab, enoblituzumab, ensituximab, epratuzumab, ertumaxomab, etaracizumab, farletuzumab, ficlatuzumab, figitumumab, flanvotumab, futuximab, galiximab, ganitumab, icrucumab, igovomab, imalumab, imgatuzumab, indusatumab, inebilizumab, intetumumab, iratumumab, isatuximab, lexatuzumab, lilotomab, lintuzumab, lirilumab, lucatumumab, lumretuzumab, margetuximab, matuzumab, mirvetuximab, mitumomab, mogamulizumab, moxetumomab, nacolomab, naptumomab, narnatumab, necitumumab, nesvacumab, nimotuzumab, nivolumab, nofetumomab, obinutuzumab, ocaratuzumab, ofatumumab, olaratumab, onartuzumab, ontuxizumab, oportuzumab, oregovomab, otlertuzumab, pankomab, parsatuzumab, pasotuxizumab, patritumab, pembrolizumab, pemtumomab, pidilizumab, pintumomab, polatuzumab, pritumumab, quilizumab, racotumomab, ramucirumab, rilotumumab, robatumumab, sacituzumab, samalizumab, satumomab, seribantumab, siltuximab, sofituzumab, tacatuzumab, taplitumomab, tarextumab, tenatumomab, teprotumumab, tetulomab, ticilimumab, tigatuzumab, tositumomab, tovetumab, tremelimumab, tucotuzumab, ublituximab, ulocuplumab, urelumab, utomilumab, vadastuximab, vandortuzumab, vantictumab, vanucizumab, varlilumab, veltuzumab, vesencumab, volociximab, vorsetuzumab votumumab, zalutumumab, zatuxima, tisotumab, zilovertamab, telisotuzumab, combinations and derivatives thereof., as well as other monoclonal antibodies targeting CAI 25, CAI 5-3, CAI 9-9, L6, Lewis Y, Lewis X, alpha fetoprotein, CA 242, placental alkaline phosphatase, prostate specific antigen, prostate specific membrane antigen, prostatic acid phosphatase, epidermal growth factor, MAGE-I, MAGE-2, MAGE-3, MAGE-4, transferrin receptor, p97, MUCI, CEA, gplOO, MARTI, IL-2 receptor, CD20, CD52, CD33, CD22, human chorionic gonadotropin, CD38, CD40, mucin, P21, MPG, Neu oncogene product, HER2, Nectin-4, tissue factor, Trop-2, FRα, ROR1, EGFR, cMET and B7-H3.

According to an embodiment, Protein may be an engineered antibody (or so called "mutant" antibody).

A particular illustrative embodiment is an antibody comprising two heavy chains and two light chains wherein 6 out of 8 interchain cysteines, which typically form the interchain disulfide bonds, were mutated into any other amino acid, either the same or different, such as lysine, arginine, tyrosine, phenylalanine, tryptophan, histidine, serine, threonine. Typically, the resulting engineered antibody comprised a single interchain disulfide bond. Illustrative examples of such engineered antibody include trastuzumab (HC-C229S, HC-C223S, LC-C214S) mutant and J591 (HC-C220S, HC-C229S, LC-C214S) mutant.

Another illustrative embodiment is an engineered antibody wherein two additional cysteine groups have been introduced into the protein sequence.

Illustrative examples of such engineered antibody include Thiomab^{®} described in US7,521,541, as well as antibodies with cysteines introduced through following point mutations: LC-V110C, HC-A114C, HC-D265C, HC-S239C, HC-S442C, HC-N325C, HC-L328C.

### The compounds of formula (II) and mixtures thereof

Ab' derives from said Protein in that it comprises two sulphur atoms that would otherwise form an interchain disulphide bridge that are reduced.

Specifically, in Ab' the Sulfur atom of each N-proximal inter(heavy chains) cysteine residue is reduced, while the remaining interchain cysteine residues are unaffected and still interchain disulfide bridges.

In Ab', the two said reduced sulphur atoms are involved in a coordination bond with said Metal atom, thus forming a group -S-M-S- that links together the two heavy chains.

According to an embodiment, the metal atom is a transition metal, in particular chosen from Zn, Co, Cd, Hg, radioactive isotopes of said atoms, or the combination thereof. Preferably, M is Zn.

According to an embodiment, Ab' is an antibody, in particular Immunoglobulin G, or a derivative thereof, where its two N-proximal sulfur atoms are reduced.

As an illustration, said complex of formula (II) may be schematically represented by the following formula:

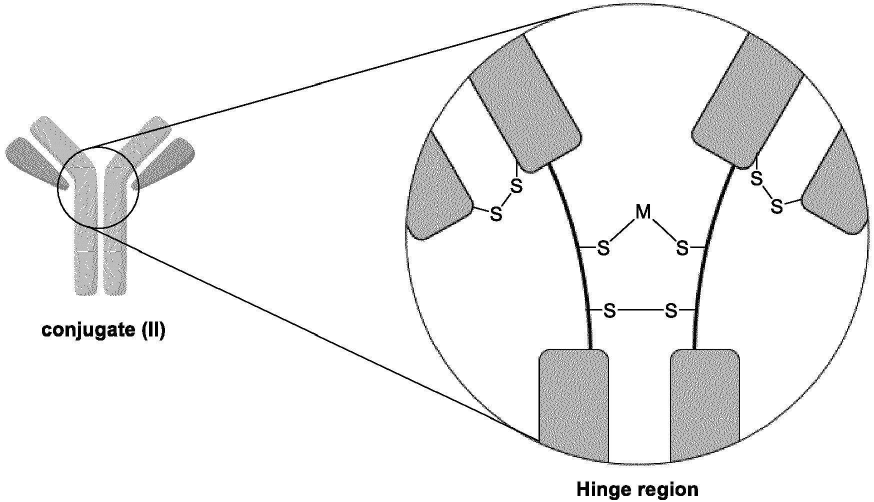
conjugate (II)
S
S
S
S
M
S
S
S
S
S
S
Hinge region

The compounds of formula (II) represent versatile key intermediates towards a diversity of antibody-drug conjugates (ADCs) of formula (III) as shown below.

### The process of preparation of the compounds of formula (II) and mixtures thereof According to an embodiment, said oxidizing agent is dehydroascorbic acid (DHAA).

According to an embodiment, the process may be carried out on an isolated compound of formula (I) or a mixture comprising the same, in particular a mixture where the compounds of formula (I) are predominantly present, as defined herein.

As an illustration, said process of preparation of said complex of formula (II) may be schematically illustrated by the following scheme:

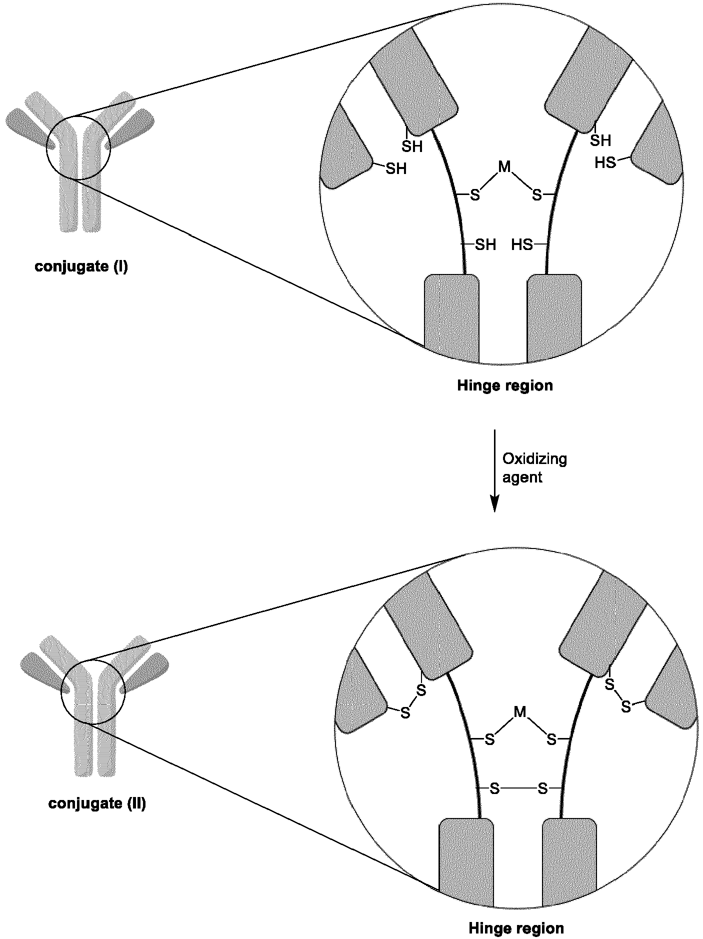
conjugate (I)
SH
SH
SH
HS
M
S
S
SH
HS
Hinge region
Oxidizing
agent
conjugate (II)
S
S
S
S
M
S
S
S
S
Hinge region

### The compounds of formula (I) and mixtures thereof

[Ab'_{Red}-M] (I)

Where Ab'_{Red} differs from Ab' as defined herein in that apart from the two reduced sulfur atoms that are involved in the coordination complex with the Metal atom, the other sulfur atoms are reduced.

More specifically, Ab'_{Red} is a protein or fragment thereof comprising two heavy chains and two light chains, each chain comprising one or more cysteine residue involved in inter chains disulfide bridges,
wherein each heavy chain comprises one N-proximal inter(heavy chains) cysteine residue that is located nearest to the N-terminal end of said heavy chain,
And wherein each Sulfur atom of each said N-proximal inter(heavy chains) cysteine residue is reduced and the two reduced Sulfur atoms form together with the M atom the following group: -S-M-S- within the coordination complex;
and the Sulfur atoms of the remaining interchain cysteine residues are reduced in the form of thiol -SH groups.

As an illustration, said complex of formula (I) may be schematically represented by the following formula:

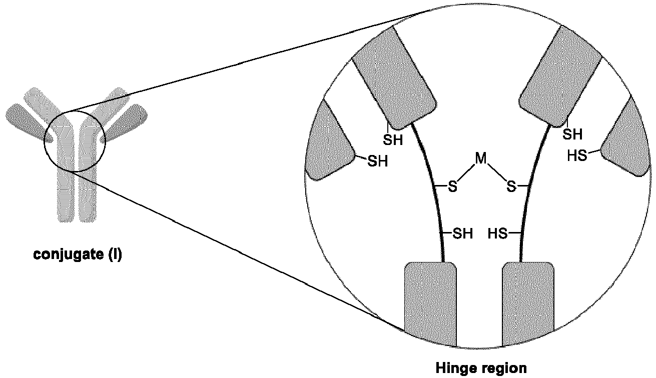
conjugate (I)
SH
SH
M
S
S
SH
HS
SH
HS
Hinge region

### The process of preparation of the complex of formula (I) and mixtures thereof

Ab is a protein or fragment thereof comprising two heavy chains and two light chains, each chain comprising one or more cysteine residue involved in inter chains disulfide bridges.

Ab may be Protein as defined above.

Ab_{Red} refers to reduced Ab, and thus differs from Ab in that all Sulfur atoms of said interchain cysteine residues are reduced and in the form of thiol -SH groups.

Said reducing agent may be for example Tris(2-carboxyethyl)phosphine hydrochloride (TCEP), dithiothreitol (DTT) or 2-mercaptoethanol (BME).

For the reducing step, preferred conditions include 37 °C, 1.5 h, 5-10 equiv. TCEP.

According to an embodiment, the metal salt may be chosen from dihalogenide metal salts, such as ZnCl₂, or Zn(OAc)₂.

Typically, the step of reacting the metal salt may be conducted in aqueous buffers, such as phosphate-buffered saline, at room temperature, over 5-10 minutes.

As an illustration, said process of preparation of said complex of formula (I) may be schematically illustrated by the following scheme:

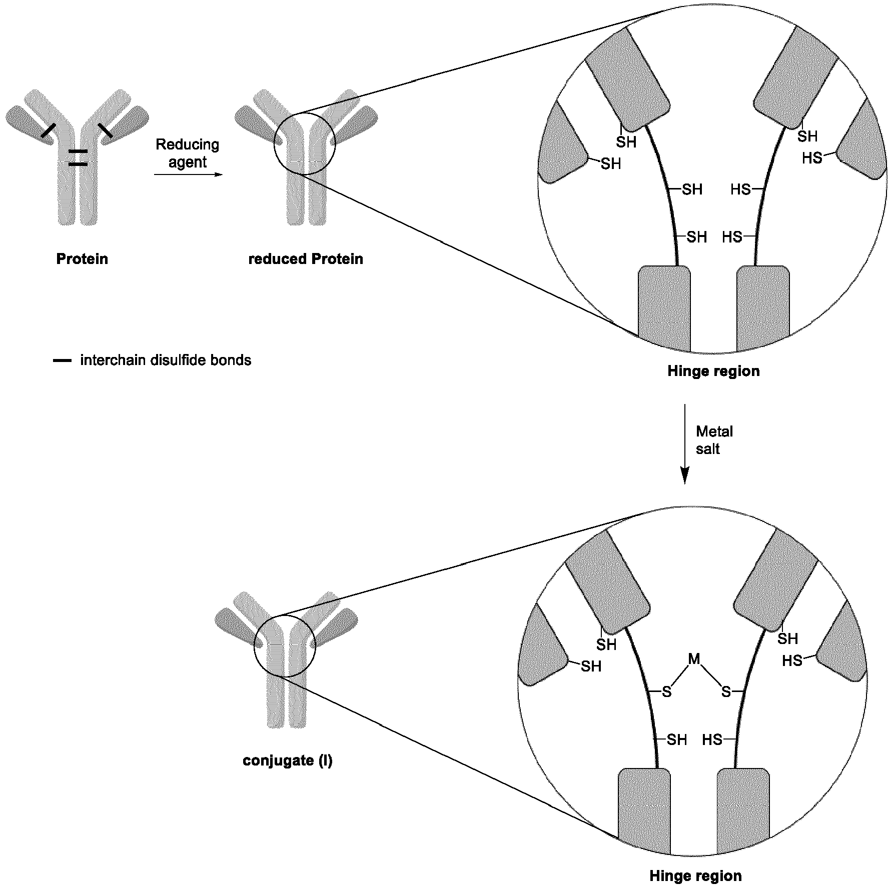
Protein
Reducing
agent
reduced Protein
— interchain disulfide bonds
SH
SH
SH
SH
SH
HS
HS
HS
Hinge region
Metal
salt
conjugate (I)
SH
SH
M
S
S
SH
HS
SH
HS
Hinge region

### The compounds of formula (III) and mixtures thereof

Compounds of formula (III) may be referred to as « antibody-drug conjugates » (ADC).

According to an illustrative embodiment, the conjugate of formula (III) may be a monoconjugate (DAR=1 ). Accordingly, L is a trivalent linker covalently binding said Payload with the two N-proximal sulfur atoms.

Said protein conjugate of formula (III) may be of formula (III-A):

Ab'-L-( Payload) (III-A)

Having a controlled drug-to-antibody ratio (DAR) of 1;
Where in formula (III-A):
   Ab' and Payload are defined as herein;
   L is a trivalent linker moiety covalently binding together the two said reduced Sulfur atoms of Ab' with Payload.

Preferably, said conjugate of formula (III-A) comprises one of the following fragments: 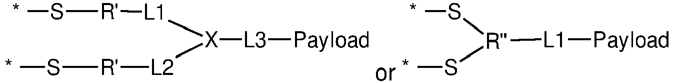
Wherein each S designates the reduced Sulfur atom of each said N-proximal inter(heavy chains) cysteine residue;
* designates the attachment of said Sulfur atom to the rest of Ab' at each heavy chain through the corresponding N-proximal inter(heavy chains) cysteine residue;
L₁, L₂ and L₃ identical or different are optional divalent Spacer moieties;
X is chosen from linear, branched or cyclic trivalent moieties, comprising 1 to 9 nitrogen atoms and from 0 to 30 carbon atoms;
R' is a group formed as a result of a reaction between a thiol and a thiol-reactive group, such as maleimide, 3-arylpropiolonitrile, bromo-maleimide, chloroacetamide, bromoacetamide, iodoacetamide, acrylamide, vinyl sulfone, pyridyl disulfide, alpha-halo carbonyl, vinyl pyridine;
R" is a group formed as a result of a reaction between two thiols and a bifunctional thiol-reactive group, such as 3,4-disubstituded maleimide, 3,3'-(phenylene)dipropiolonitrile, dibromopyridazinedione, bissulfone, allyl sulfone, 3-bromomaleimide or arsenous acid.

As an illustration, said conjugate of formula (III-A) may be schematically represented by the following formula (III-A1):

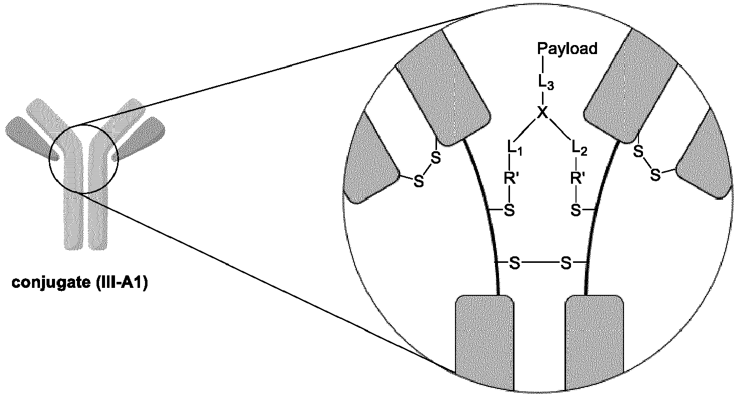
conjugate (III-A1)
Payload
L3
X
L1
L2
R'
R'
S
S
S
S
S
S
S
S

Where X, L₁, L₂ and L₃ are defined as above.

As a further illustration, said conjugate of formula (III-A) may be schematically represented by the following formula (III-A2):

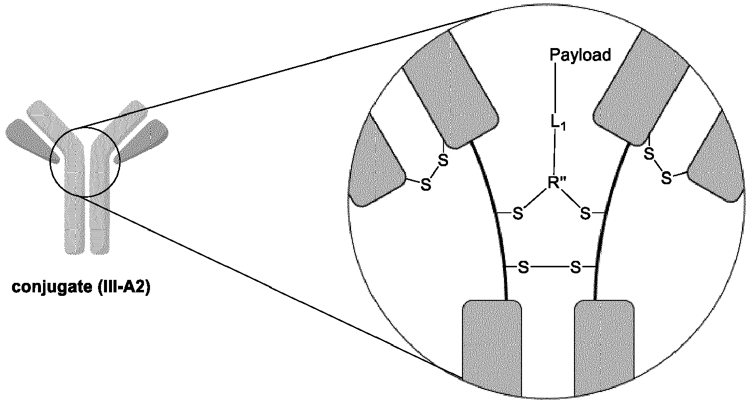
Payload
L1
S
S
R"
S
S
S
S
S
S
S
S
conjugate (III-A2)

Where X, L, are defined as above.

According to another illustrative embodiment, the conjugate of formula (III) may be an even multiple conjugate (DAR=2m).

Said protein conjugate of formula (III) may be of formula (III-B):

Ab'-(L-(Payload)ₘ)₂ (III-B)

Having a controlled drug-to-antibody ratio (DAR) of 2m;
Where in formula (III-B):
   m is as defined above, preferably 1, 2, 3, 4, 5 or 6;
   Ab' and Payload are defined as herein;
   Each of the two L is di-, tri-, tetra-, penta-, hexa- or hepta-valent linear or branched linker moiety covalently binding one of said reduced Sulfur atoms of said N-proximal inter(heavy chains) cysteine residue of Ab' with m Payload(s).

It is herein understood that when m > 1, then each Payload can be the same or different.

Preferably, said conjugate of formula (III-B) comprises two following fragments:

* -S-R'-L4-(Payload)ₘ

Wherein
m and Payload are defined as herein;
S designates the reduced Sulfur atom of each of the two said N-proximal inter(heavy chains) cysteine residues, respectively;
* designates the attachment of said Sulfur atom to the rest of Ab' at each heavy chain through the corresponding N-proximal inter(heavy chains) cysteine residue;
Each L₄ identical or different is an optional linear or branched Spacer moiety;
R' is a group formed as a result of a reaction between the reduced thiol group of Ab' and a thiol-reactive group, such as maleimide, 3-arylpropiolonitrile, bromo-maleimide, chloroacetamide, bromoacetamide, iodoacetamide, acrylamide, vinyl sulfone, pyridyl disulfide, alpha-halo carbonyl, vinyl pyridine.

As an illustration, said conjugate of formula (III-B) may be schematically represented by the following formula:

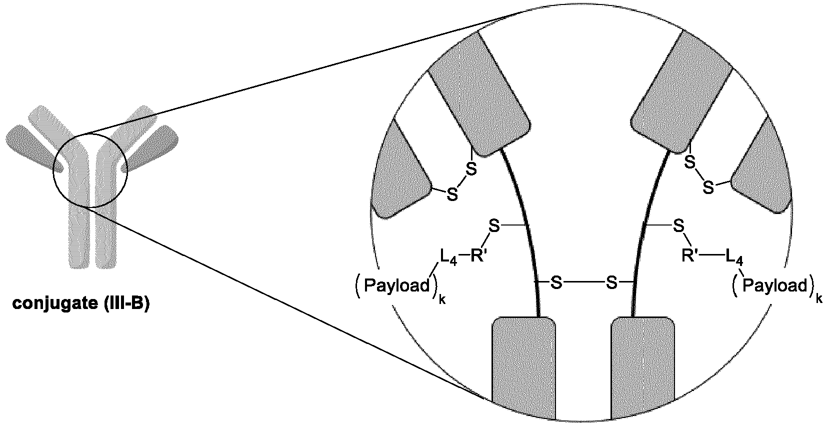
conjugate (III-B)
S
S
S
S
S
S
L4—R'
R'—L4
(Payload)k
(Payload)k
S—S

Where R', L₄ are defined as above and k is defined as m above.

According to another illustrative embodiment, the conjugate of formula (III) may be a multiple conjugate (DAR=m).

Said protein conjugate of formula (III) may be of formula (III-C):

T-Ab'-L-(Payload)ₘ (III-C)

Having a controlled drug-to-antibody ratio (DAR) of m;
Where in formula (III-C):
   m, Ab' and Payload are as defined herein;
   L is di-, tri-, tetra- or penta-valent linear or branched linker moiety covalently binding one of said reduced Sulfur atoms of the two N-proximal inter(heavy chains) cysteine residues of Ab' with m Payload(s); and
   T is defined as herein and is covalently bound to Ab' through the other one of said reduced Sulfur atoms of the two N-proximal inter(heavy chains) cysteine residues of Ab'.

Preferably, said conjugate of formula (III-C) comprises each of the two following fragments:

* -S-R'-L4-(Payload)ₘ

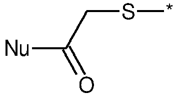

Wherein
Payload and m are defined as herein;
Each S designates one reduced Sulfur atom of each of the two said N-proximal inter(heavy chains) cysteine residues;
Each * designates the attachment of said Sulfur atom to the rest of Ab' at each heavy chain through the corresponding N-proximal inter(heavy chains) cysteine residue;
Each L₄ identical or different is an optional linear or branched Spacer moiety;
R' is a group formed as a result of a reaction between the reduced thiol group of Ab' and a thiol-reactive group, such as maleimide, 3-arylpropiolonitrile, bromo-maleimide, chloroacetamide, bromoacetamide, iodoacetamide, acrylamide, vinyl sulfone, pyridyl disulfide, alpha-halo carbonyl, vinyl pyridine;
Nu is a nucleophilic moiety chosen from OH, -NH₂, -NH-NH₂, -NH-OH, -NH-R₁, -NR₁R₂, Where R₁, R₂ and R₃ are chosen from -CH₂-CONH₂, -CH₂-COOH, C1-C12 alkyl, C6-C12 aryl, C5-C12 heteroaryl; -(CH₂-CH₂₋O-)ᵣ-CH₃ groups; in which r is an integer ranging from 1 to 24.

As an illustration, said conjugate of formula (III-C) may be schematically represented by the following formula:

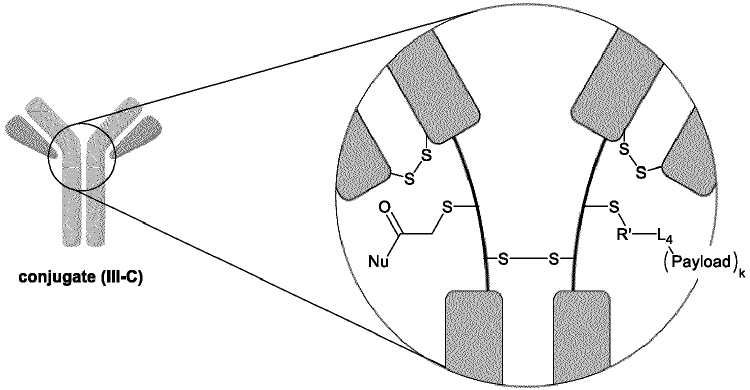
conjugate (III-C)
S
S
S
S
O
S
S
R'
L4
Nu
S
S
(Payload)k

Where R', L₄ are defined as above and k is defined as m above.

In the formulae (III-A1), (III-A2), (III-B) and/or (III-C) above and below:
According to an embodiment, the optional L₁, L₂ and L₃ spacers may consist of one or multiple successive units selected from the group consisting of: a linear or branched, saturated or unsaturated, C1-C60 alkylene group optionally interrupted and/or terminated on one or both sides by one or more chemical groups selected from -O-, -S-, -S(O)-, -SO₂-, -O-P(O)(OH)-O-, -C(O)-, -NH-, -NMe-, -C(O)NH-, -NHC(O)-, -NH-C(O)-NH, -O-C(O)-NH-, -O-C(O)-O-; C3-C8 cycloalkylene; C3-C8 heterocyclylene; 5 to 12 membered heteroarylene; C6-C12 arylene; amino acids; polypeptides; glycosylene; -(CH₂-CH₂-O-)ᵣ- groups; in which r is an integer ranging from 1 to 24 and/or optionally substituted by one or more of OH, CN, NH₂, CF₃, NHCONH₂, CONH₂, COOH, =O, NH₂, glycosyl, C1-C12 alkyl, C3-C8 cycloalkyl, C6-C12 aryl, C5-C12 heteroaryl, -(CH₂-CH₂-O-)ᵣ-CH₃.

According to an embodiment, R' may include the following groups:

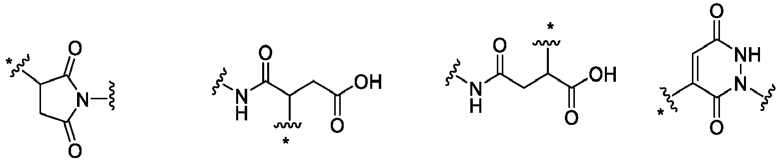

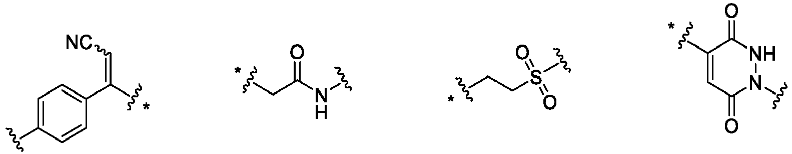

According to an embodiment, R" may include the following groups: 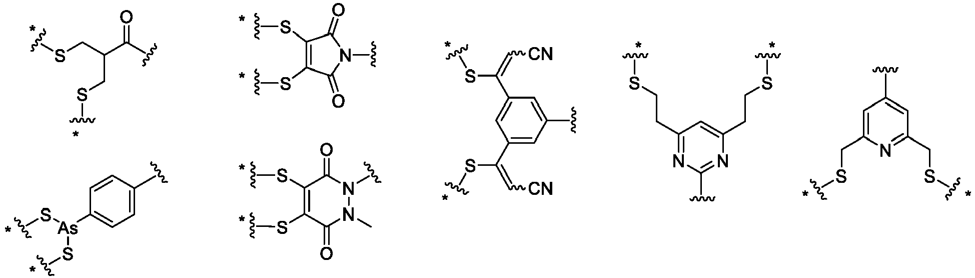 where
Y may be Br, CI, I, SPh, SCH₂CH₂OH.

According to a particular embodiment, X may be chosen from the following groups:

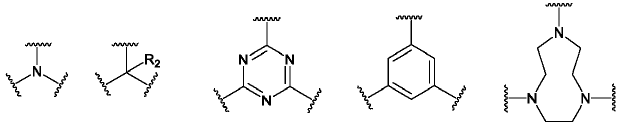

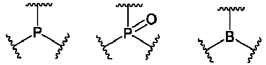

Where R₂ is chosen from H, OH, CN, NH₂, CF₃, NHCONH₂, CONH₂, COOH, =O, NH₂, C1-C12 alkyl, C6-C12 aryl, C5-C12 heteroaryl; -(CH₂-CH₂-O-)ᵣ-CH₃ groups; in which r is an integer ranging from 1 to 24.

According to an embodiment, the optional L₄ spacer may consist of one or multiple linear or branched units, which links interchain cysteine residue of the antibody with one or multiple Payload molecules.

Linear units of the L₄ include: saturated or unsaturated, C1-C60 alkylene group optionally interrupted and/or terminated on one or both sides by one or more chemical groups selected from -O-, -S-, -S(O)-, -SO₂-, -O-P(O)(OH)-O-, -C(O)-, -NH-, -NMe-, -C(O)NH-, -NHC(O)-, - NH-C(O)-NH, -O-C(O)-NH-, -O-C(O)-O-; C3-C8 cycloalkylene; C3-C8 heterocyclylene; 5 to 12 membered heteroarylene; C6-C12 arylene; amino acids; polypeptides; glycosylene; - (CH₂-CH₂-O-)ᵣ- groups; in which r is an integer ranging from 1 to 24 and/or optionally substituted by one or more of OH, CN, NH₂, CF₃, NHCONH₂, CONH₂, COOH, =O, NH₂, glycosyl, C1-C12 alkyl, C3-C8 cycloalkyl, C6-C12 aryl, C5-C12 heteroaryl, -(CH₂-CH₂-O-)ᵣ-CH₃.

Branched units of the L₄ may be chosen from branched or cyclic trivalent or tetravalent moieties, comprising 1 to 9 nitrogen atoms and from 0 to 30 carbon atoms, including the following groups:

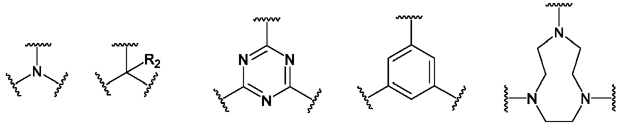

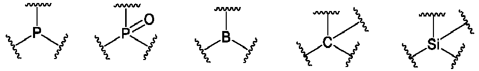

### Payload

Payload may be chosen from drug or imaging agents, oligonucleotides, chelating ligands capable of complexing with radionuclides, cytotoxic drugs and antineoplastic agents. According to an embodiment, Payload may be chosen from drugs, such as cytotoxic drugs and antineoplastic agents.

Suitable drugs include in particular:
- dolastatins such as MMAE, MMAF, MMAD;
- maytansins such as DM1 and DM4;
- antracyclins such as doxorubicin, nemorubicin and PNU-159682;
- calicheamicins;
- duocarymycins such as CC-1065 and duocarmycin A;
- pyrrolobenzodiazepines;
- pyrrolobenzodiazepine dimers;
- indolino-benzodiazepines;
- indolino-benzodiazepine dimers;
- amanitins such as α-amanitin, β-amanitin, γ-amanitin, ε-amanitin;
- irinotecan derivatives;
- exatecan derivatives;
- exotoxins such as diphtheria toxin, shiga toxin, or subunits thereof;
in particular:
- dolastatins such as MMAE, MMAF, MMAD;
- maytansins such as DM1 and DM4;
- antracyclins such as doxorubicin, nemorubicin and PNU-159682;
- calicheamicins;
- duocarymycins such as CC-1065 and duocarmycin A;
- pyrrolobenzodiazepines;
- pyrrolobenzodiazepine dimers;
- indolino-benzodiazepines;
- indolino-benzodiazepine dimers;
- amanitins such as α-amanitin, β-amanitin, γ-amanitin, ε-amanitin; and
- irinotecan derivatives;
- exatecan derivatives.
- vincristine derivatives.

Suitable oligonucleotides include silencing RNA, microRNA, antisense oligonucleotides, DNA, or RNA oligomers. Typically they are between 10 and 5000 nucleotides in length, more particularly, between 20 and 200.

Imaging agents include detection agents, biological markers, tracing agents, such as fluorophores, dyes, radioactive tracers.

The -(L-(Payload)ₘ)ₙ fragments present in compounds of formula (III) may be diversely chosen and are not limited according to the invention. Many such fragments have been described and developed in existing conjugates.

Representative known fragments for -S-L-(Payload) include:

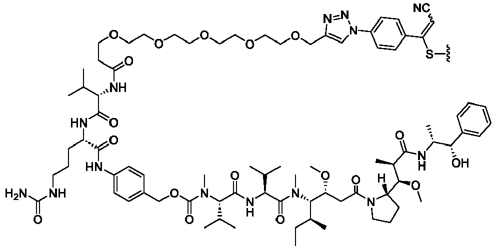

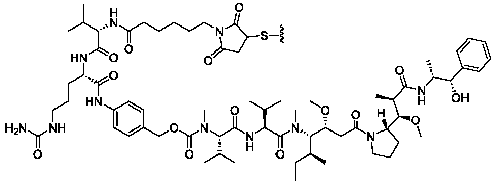

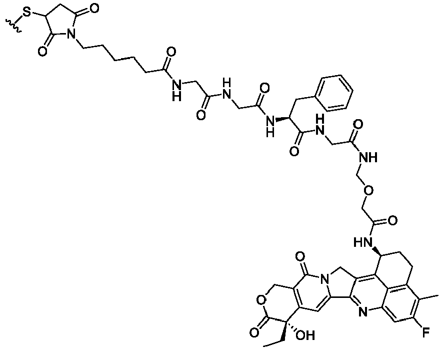

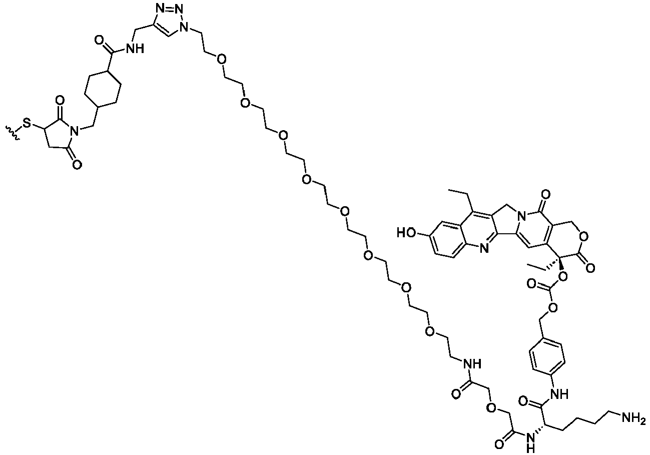

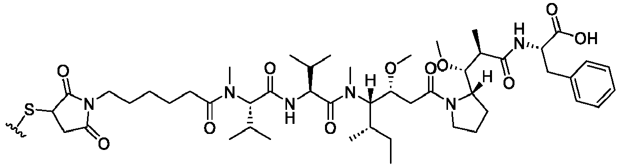

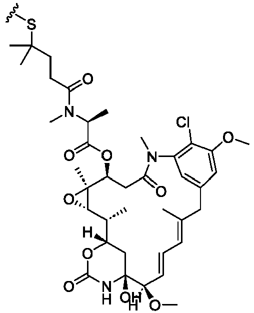

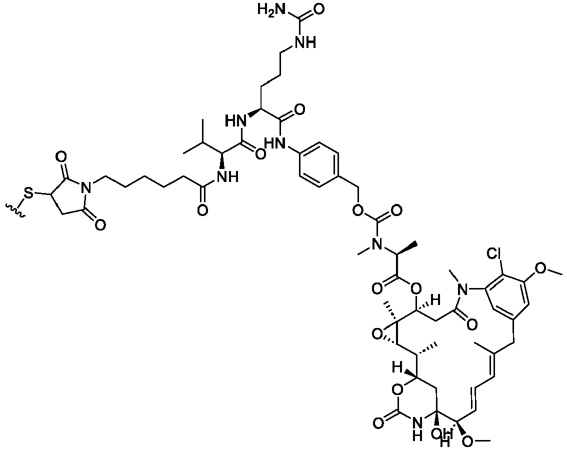

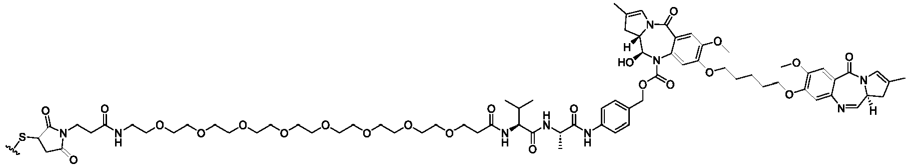

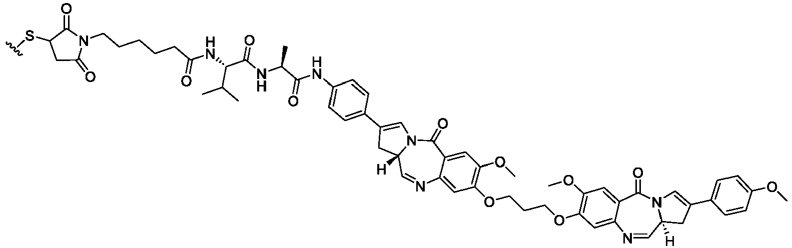

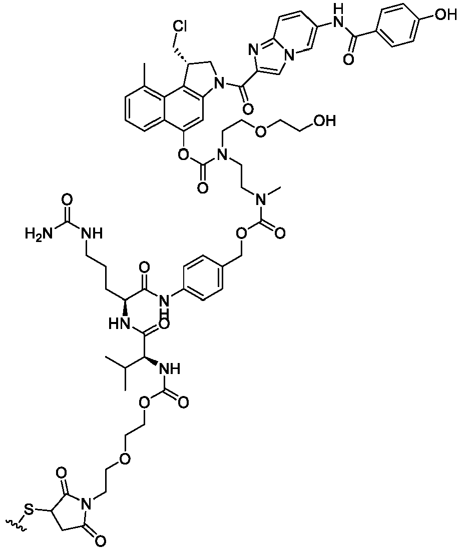

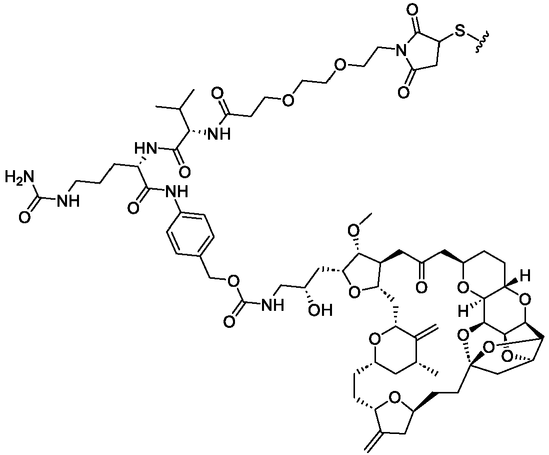

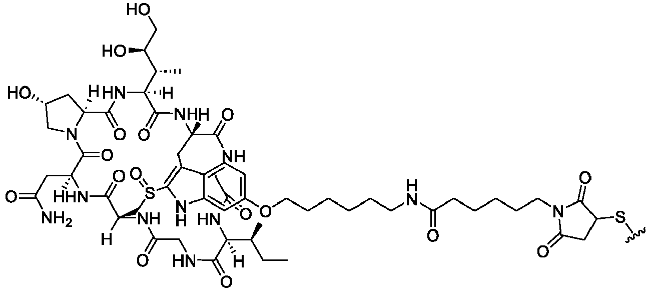

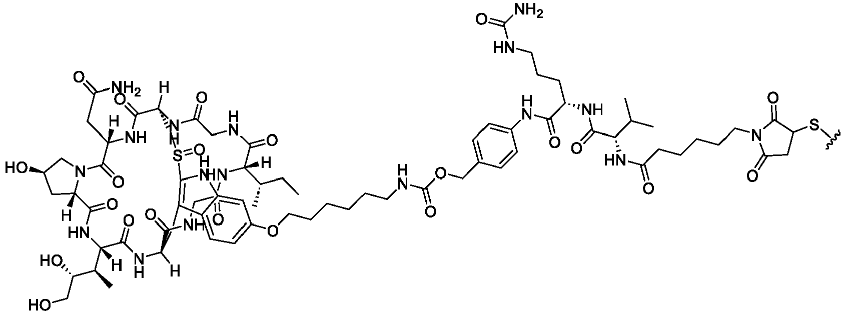

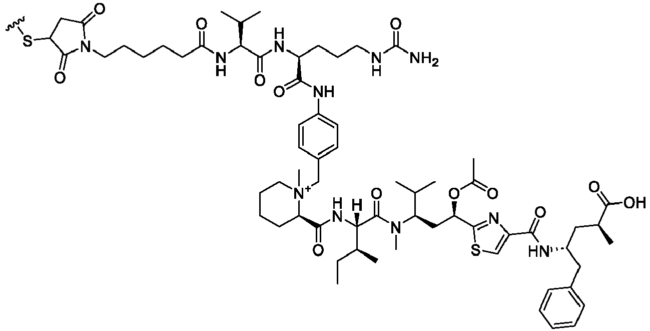

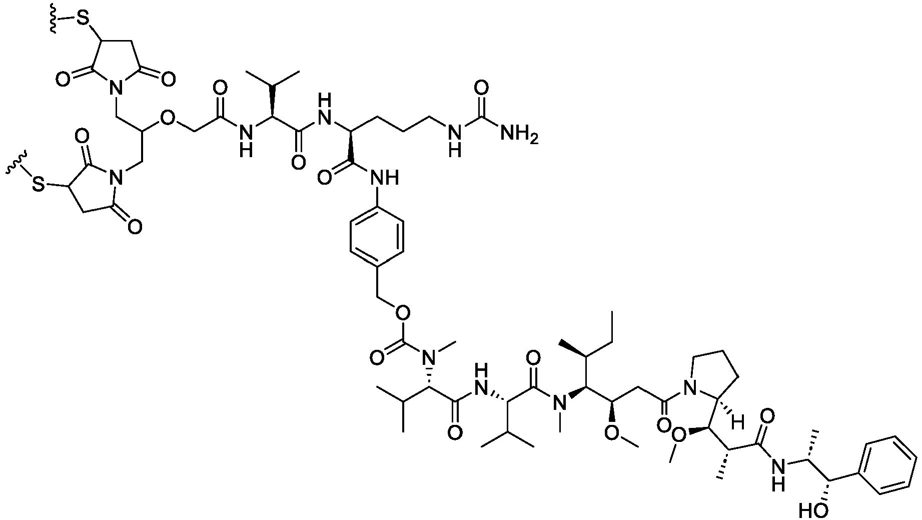

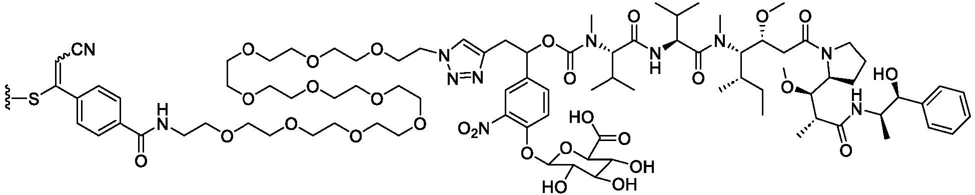

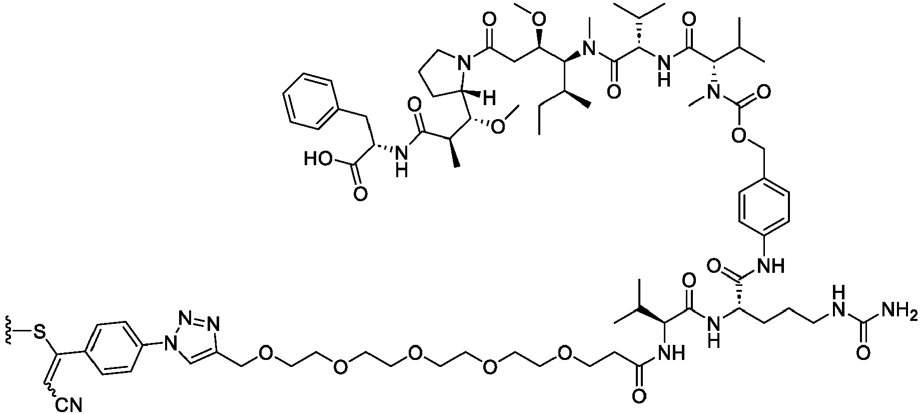

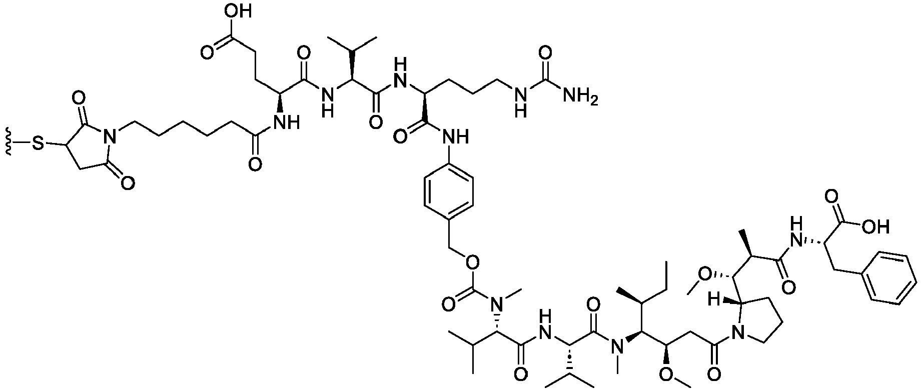

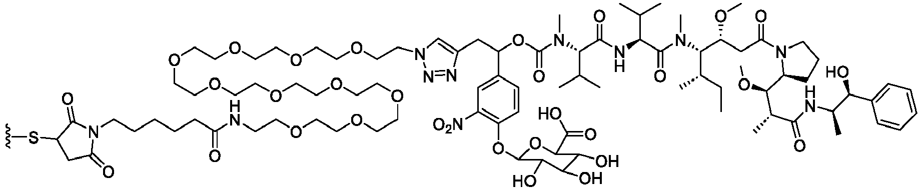

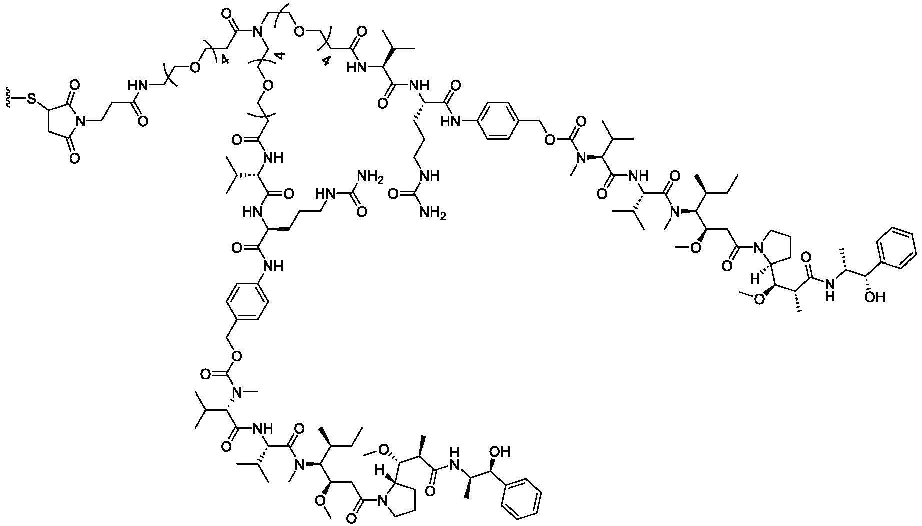

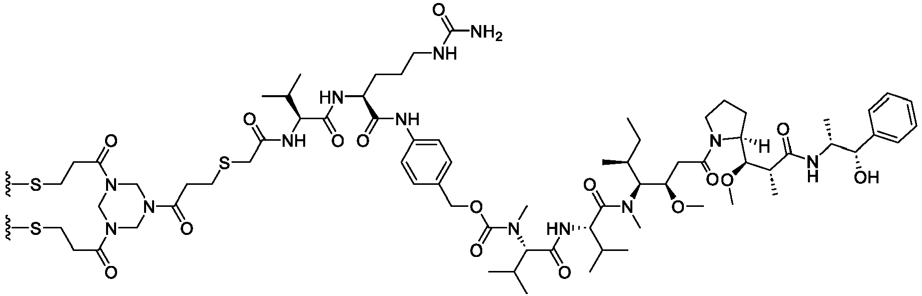

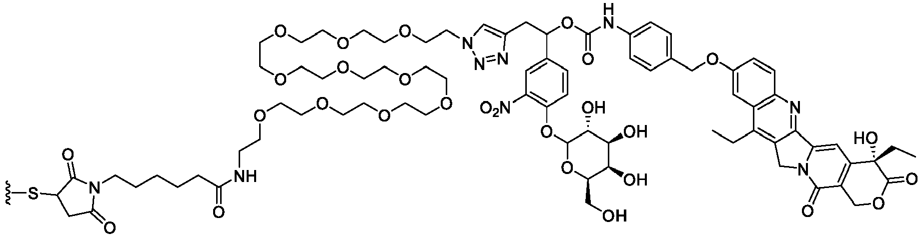

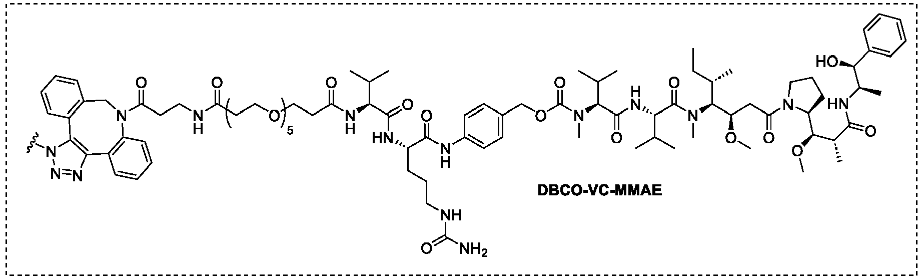

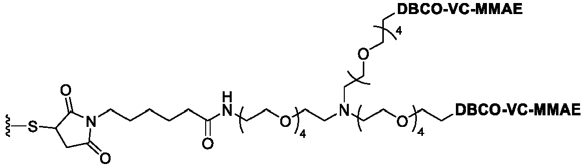

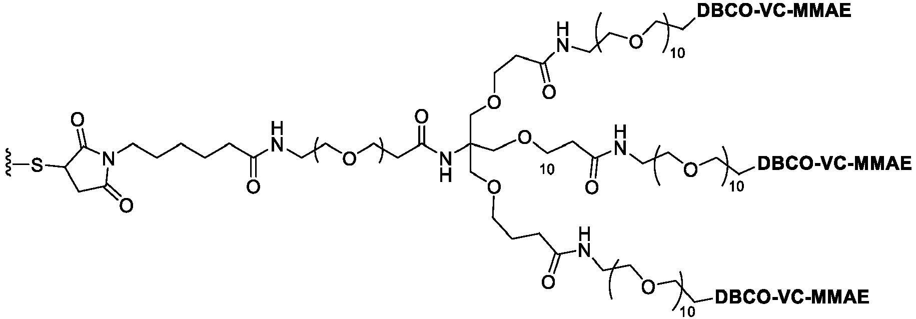

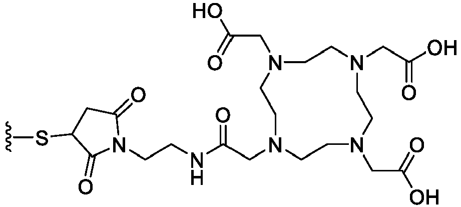

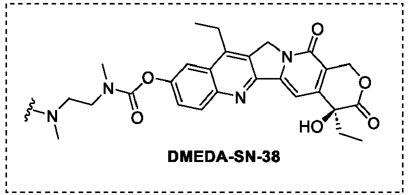

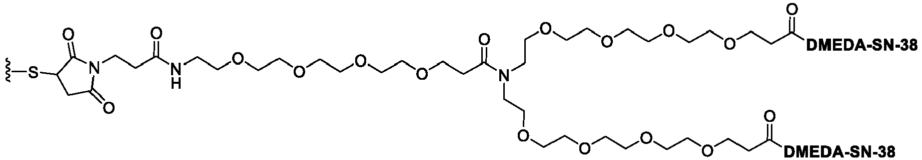

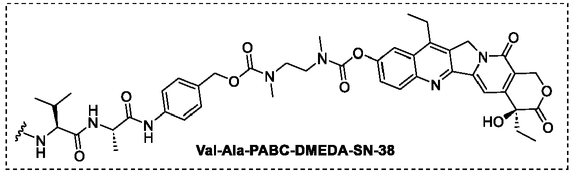

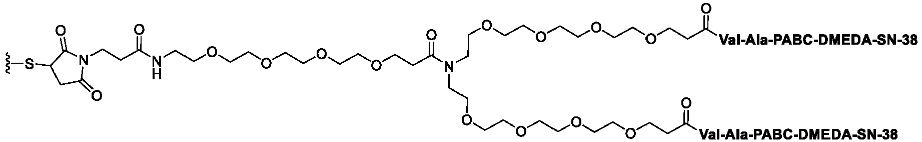

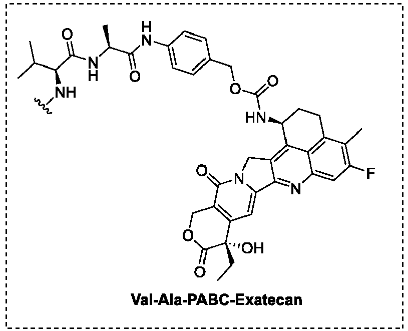

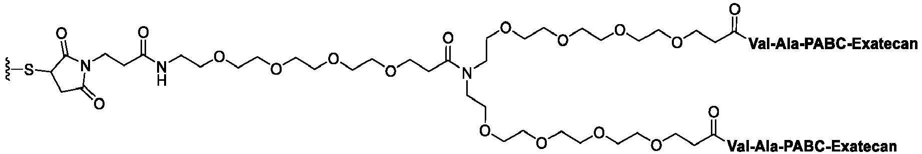

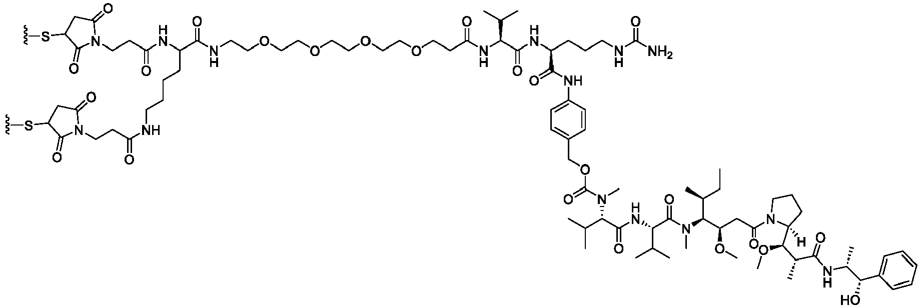

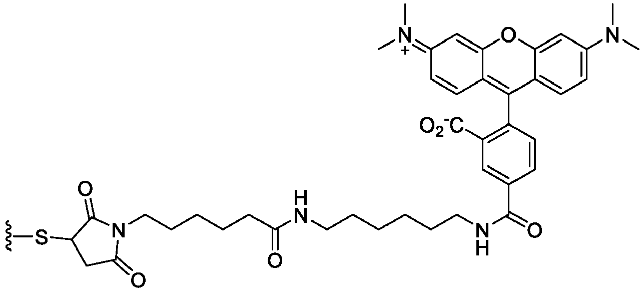

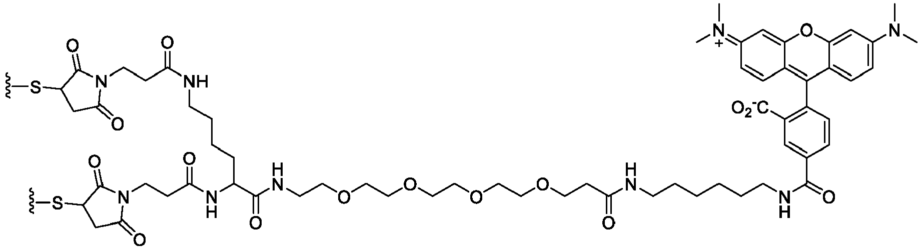

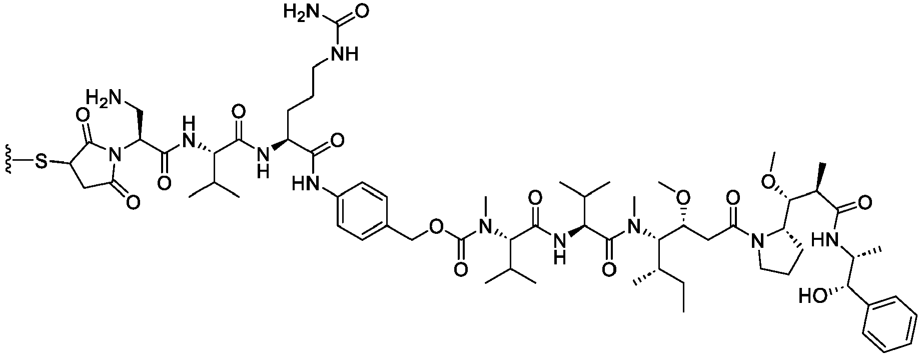

### The process of preparation of the compounds of formula (III) or mixtures thereof

The process may typically comprises reacting a compound of formula (II) with a metal complexing agent so as to form a de-complexed compound.

Said metal complexing agent may typically be chosen from ethylenediaminetetraacetic acid (EDTA), diethylenetriaminepentaacetic acid (DTPA), nitrilotriacetic acid (NTA).

The process also comprises the coupling of said decomplexed compound with a conjugation agent. Said conjugation agent may be chosen from thiol-reactive compounds.

Said de-complexing step and conjugating step may be conducted separately in sequence or as a one pot reaction.

According to an embodiment, all steps are preferably conducted in a buffered reacting medium, selected from the group comprising Hepes, Histidine buffer, PBS, or MES, having a pH value comprised between 5.5 and 8, and at a temperature of about -10°C to 37°C, for example, at about 0°C to 20°C.

Typically, reactions may be conducted in conditions compatible with Protein, such as pH comprised between 7 and 9, preferably between 7 and 8, at a temperature comprised between 20 and 40°C, preferably at room temperature.

Typically, the reaction may be conducted in a buffered medium such as TBS, PPB, PBS, HEPES medium. Reaction time may be comprised between 1 and 10 hours, preferably between 1 and 5 hours.

The concentration of Protein in the reactional mixture may be adjusted; advantageously it may be comprised between 6.0 and 8.0 g/L.

The nature of the reagents and experimental conditions of the process may depend on the degree of conjugation that is desired.

As an illustration:
The process of preparation of a conjugate of formula (III-A) may be schematically represented by the following scheme:

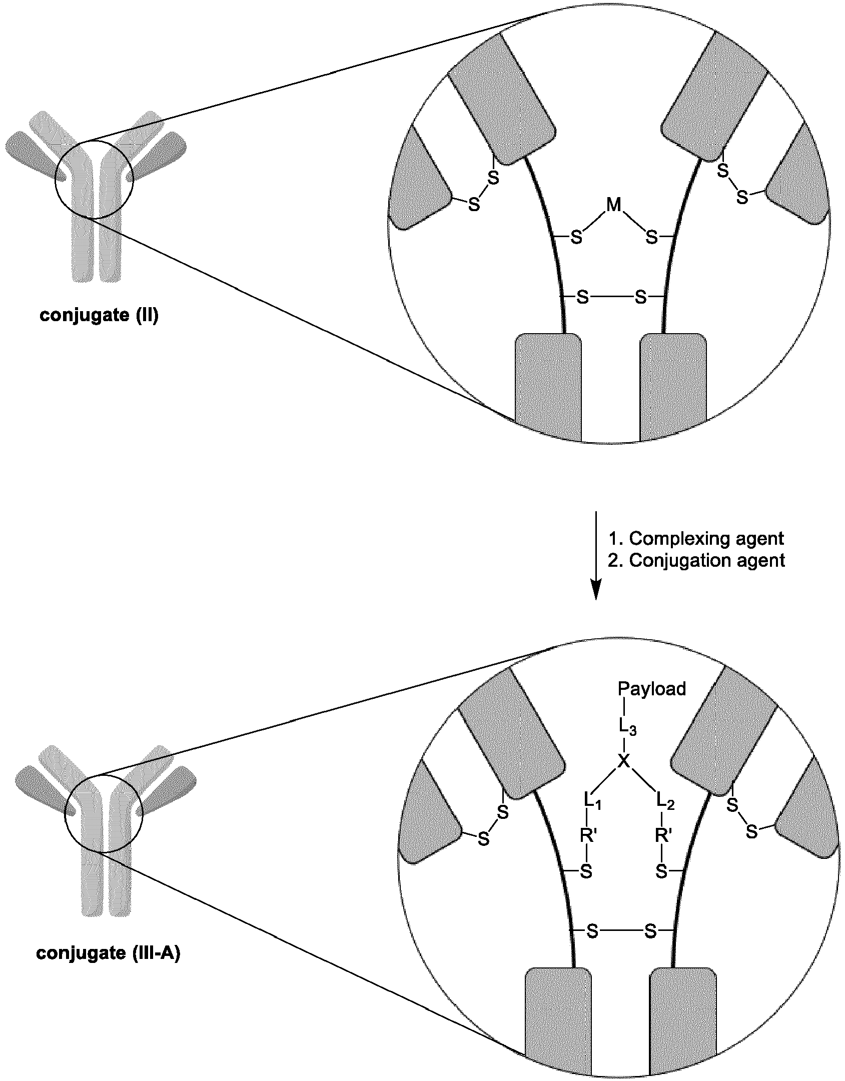
conjugate (II)
S
S
S
S
M
S
S
S
S
1. Complexing agent
2. Conjugation agent
conjugate (III-A)
Payload
L3
X
L1
L2
S
S
R'
R'
S
S
S
S
S
S

Where L₁, L₂, L₃, R', X and Payload are defined as above.

The process of preparation of a conjugate of formula (III-B) may be schematically represented by the following scheme:

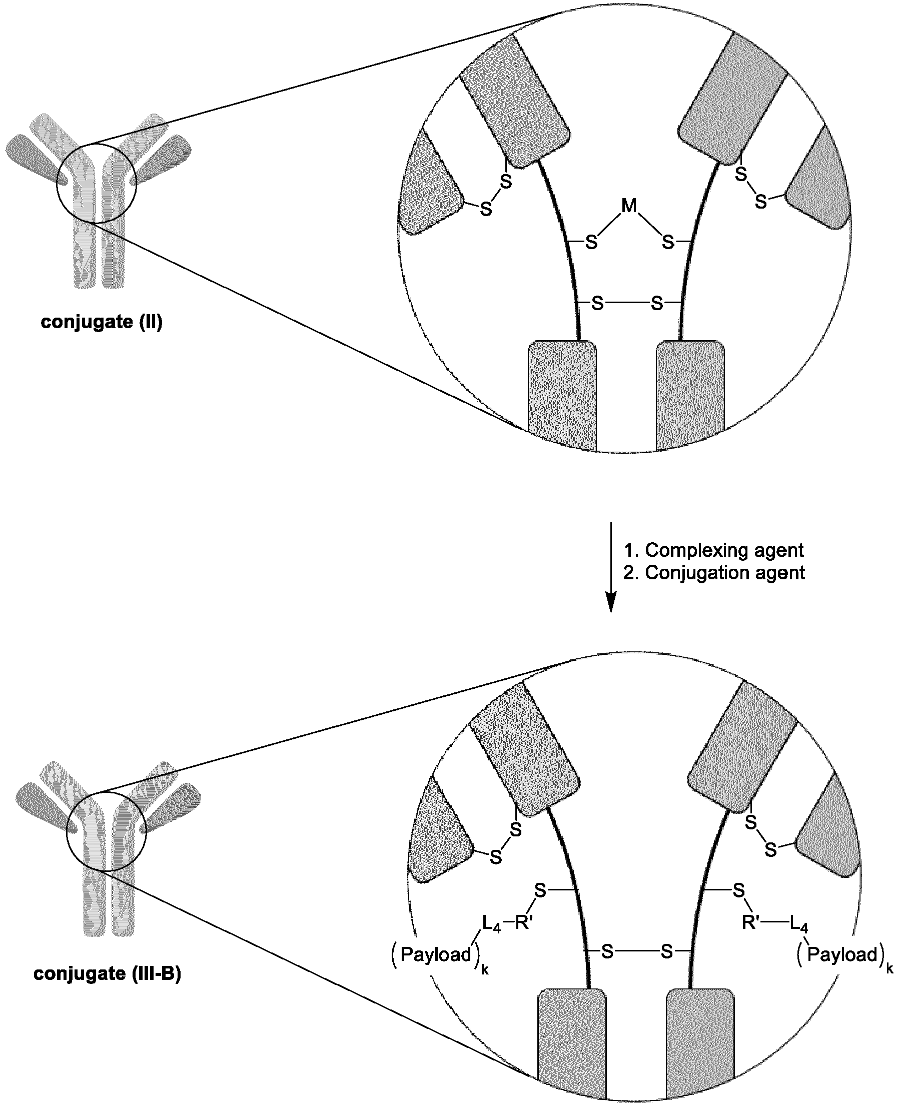
conjugate (II)
S
S
M
S
S
S
S
S
S
1. Complexing agent
2. Conjugation agent
conjugate (III-B)
S
S
S
S
S
L4—R'
R'—L4
(Payload)k
S
S
(Payload)k

Where k is defined as m above, and Payload and R' are defined as above.

The process of preparation of a conjugate of formula (III-C) may be schematically represented by the following scheme:

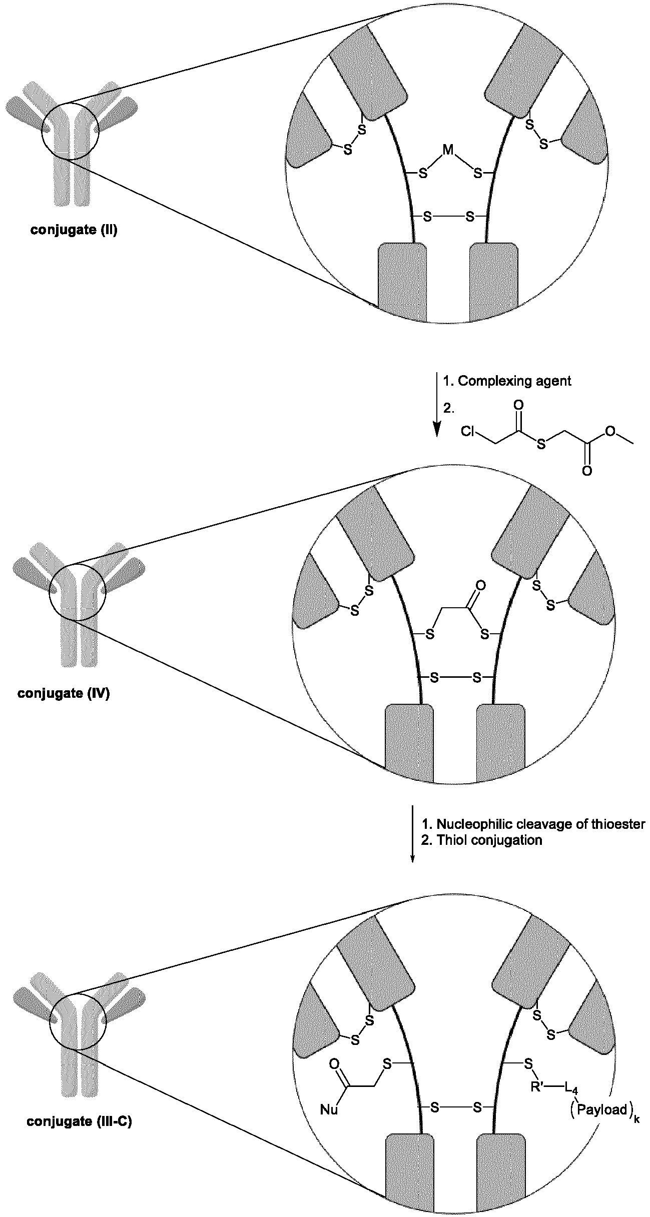
conjugate (II)
S
S
S
M
S
S
S
S
S
S
S

1. Complexing agent
2.
O
Cl
S
O
O

conjugate (IV)
S
S
O
S
S
S
S
S
S

1. Nucleophilic cleavage of thioester
2. Thiol conjugation

conjugate (III-C)
S
S
O
S
S
S
S
S
Nu
R'
L4
(Payload)k

Where k is defined as m above, and Payload, Nu and R' are defined as above.

### The mixtures

The above compounds of formula (I), (II) or (III) may be obtained or present in a mixture.

In said mixtures, the compounds of formula (I), (II) or (III), respectively, are present in major amounts.

According to an embodiment, said mixture comprises at least 50% in number, preferably at least 60%, more preferably at least 70%, still more preferably at least 80% of compounds of formula (I), (II) or (III), respectively.

In said mixtures, the compounds of formula (I), (II) or (III) are present alongside other compounds, such as side products, including different regioisomers where the coordination and/or conjugations has occurred at different locations.

Regioisomers may be formed because Protein generally comprises a number of cysteine residues that are available for reduction, coordination and subsequent conjugation. Nevetheless, it has been demonstrated that these regioisomers are formed in minor amounts as the N-proximal cysteine residues are predominantly coordinated according to the invention.

In the sense of the invention, the term "regioisomer" thus refers to isomeric coordination complexes or conjugates, where the site of reduction of sulfur atoms with cysteine residues may vary on the backbone of Protein.

The mixtures comprising the compound of formula (III) may also comprise conjugates having different -(L-(Payload)ₘ)ₙ fragments, including conjugates having different drug-antibody degree.

As discussed above, the conjugates of the invention may thus be "heterogeneous" in some extent, although enriched in one site-specific conjugate.

Therefore, in the sense of the invention, the mixtures comprising the compounds of formula (I), (II) or (III) alongside other compounds are also encompassed by the present invention.

If desired, the desired compounds of formula (I), (II) or (III) may be isolated by known techniques, such as hydrophobic interaction chromatography (HIC).

### The therapeutical use of the compounds of formula (III)

According to a particular object, the present invention concerns a pharmaceutical composition comprising a conjugate of formula (III) or mixture thereof as defined above and one or more pharmaceutically excipient.

According to a still further object, the present invention also concerns a conjugate of formula (III) or the mixture thereof as defined above, where Payload is a cytotoxic drug or antineoplastic agent, or an imaging agent for use for treating, preventing and/or diagnosing a condition or disorder chosen from the group consisting in tumor, cancer, autoimmune disease, or infectious disease in a subject.

According to a further object, the present invention also concerns a method of treating, preventing and/or diagnosing a condition or disorder chosen from the group consisting in tumor, cancer, autoimmune disease, or infectious disease in a patient in the need thereof, comprising administering a conjugate of formula (III) as defined above to said patient.

The identification of those subjects who are in need of treatment of herein-described diseases and conditions is well within the ability and knowledge of one skilled in the art. A clinician skilled in the art can readily identify, by the use of clinical tests, physical examination, genetic tests and medical/family history, those subjects who are in need of such treatment.

The therapeutically effective amount of conjugates of formula (III) can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances. The amount of conjugates of formula (II) which is required to achieve the desired biological effect, will vary depending upon a number of factors, including the dosage of the conjugates to be administered, the chemical characteristics (e.g. hydrophobicity) of the compounds employed, the potency of the compounds, the type of disease, the diseased state of the patient and the route of administration.

"Pharmaceutically" or "pharmaceutically acceptable" refer to molecular entities and compositions that do not produce an adverse, allergic or other untoward reaction when administered to an animal, or a human, as appropriate.

As used herein, "pharmaceutically acceptable excipient" includes any diluents, adjuvants, excipients, or vehicles, such as preserving agents, fillers, disintegrating agents, wetting agents, emulsifying agents, suspending agents, solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

In the context of the invention, the term "treating" or "treatment", as used herein, means reversing, alleviating, inhibiting the progress of, or preventing the disorder or condition to which such term applies, or one or more symptoms of such disorder or condition. "Therapeutically effective amount" means an amount of a compound/medicament according to the present invention effective in producing the desired therapeutic effect. According to the invention, the term "patient", or "patient in need thereof, is intended for a human or non-human mammal affected or likely to be affected with a neuropsychological disorder. Preferably, the patient is a human.

The compositions may conveniently be administered in unit dosage form and may be prepared by any of the methods well known in the pharmaceutical art, for example, as described in Remington: The Science and Practice of Pharmacy, 20th ed.; Gennaro, A. R., Ed.; Lippincott Williams & Wilkins: Philadelphia, PA, 2000. Pharmaceutically compatible binding agents and/or adjuvant materials can be included as part of the composition. Oral compositions will generally include an inert diluent carrier or an edible carrier.

### EXAMPLES

### General procedure A for the preparation of conjugate (I) with Zn as M

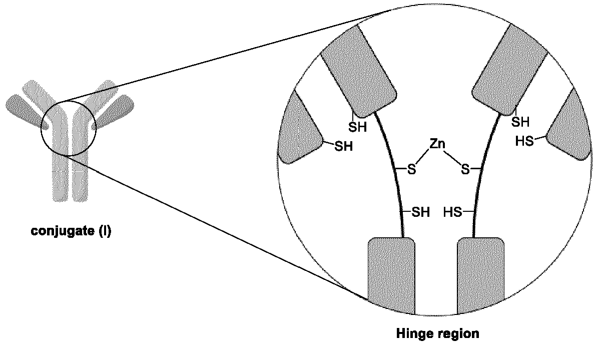
conjugate (I)
SH
SH
Zn
S
S
SH
HS
SH
HS
Hinge region

To a solution of Protein (100 µL, 40 µM in PBS pH 7.4) were added TCEP·HCI (10 mM water, 2.4 µL, 6 equiv.). The mixture was incubated at 37°C for 2 hours, followed by cooling to 20°C. Subsequently, a solution of Zn(OAc)₂ (4 mM in water, 1.4 µL, 1.4 equiv.) was added to the mixture and the mixture was incubated at 20°C for 5 minutes. The resulting conjugate (I) was purified using Bio-Spin^{®} P-30Gel Columns (Bio-Rad) which had been equilibrated with PBS buffer (1X, pH 7.4).

### General procedure B for the preparation of conjugate (II) with Zn as M

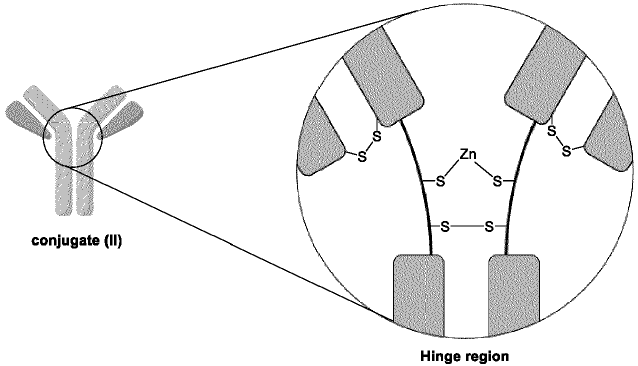
conjugate (II)
S
S
Zn
S
S
S
S
S
S
Hinge region

To a solution of Protein (100 µL, 40 µM in PBS pH 7.4) were added TCEP·HCI (10 mM water, 2.4 µL, 6 equiv.). The mixture was incubated at 37°C for 2 hours, followed by cooling to 20°C. Subsequently, a solution of Zn(OAc)₂ (4 mM in water, 1.4 µL, 1.4 equiv.) was added to the mixture and the mixture was incubated at 20°C for 5 minutes to allow for complexation of Zn²⁺ with the protein. To the resulting mixture was added dehydroascorbic acid (DHAA, 10 mM in DMSO, 6 µL, 15 equiv.) and the mixture was incubated at 20°C for 1 hour. The resulting conjugate (II) was purified using Bio-Spin^{®} P-30Gel Columns (Bio-Rad) which had been equilibrated with PBS buffer (1X, pH 7.4).

### General procedure C for the preparation of conjugate (III-A1)

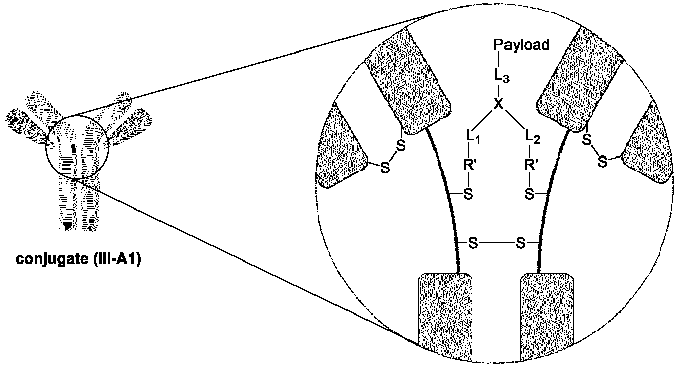
Payload
L3
X
L1 L2
R' R'
S S
S S
S S
S S
conjugate (III-A1)

To a solution of Protein (100 µL, 40 µM in PBS pH 7.4) were added TCEP·HCl (10 mM in water, 2.4 µL, 6 equiv.). The mixture was incubated at 37°C for 2 hours, followed by cooling to 20°C. Subsequently, a solution of Zn(OAc)₂ (4 mM in water, 1.4 µL, 1.4 equiv.) was added to the mixture, followed by the addition of dehydroascorbic acid (DHAA, 10 mM in DMSO, 6 µL, 15 equiv.). The resulting mixture was incubated at 20°C for 1 hour, then purified using Bio-Spin^{®} P-30Gel Column (Bio-Rad) equilibrated with PBS (1X, pH 7.4) and cooled to 4°C. To the resulting mixture was added the solution of Payload (10 mM in DMSO, 1.2 µL, 3 equiv.) followed by EDTA (0.5 M in water, 0.5 µL, pH 8.0). The mixture was incubated until completion of the conjugation reaction (e.g. 1.5 hours at 4°C for maleimide-based payloads, 16h at 20°C for APN-based payloads). The resulting conjugate (III-A1) was purified using Bio-Spin^{®} P-30Gel Column (Bio-Rad) equilibrated with PBS (1X, pH 7.4).

### General procedure D for the preparation of conjugate (III-B)

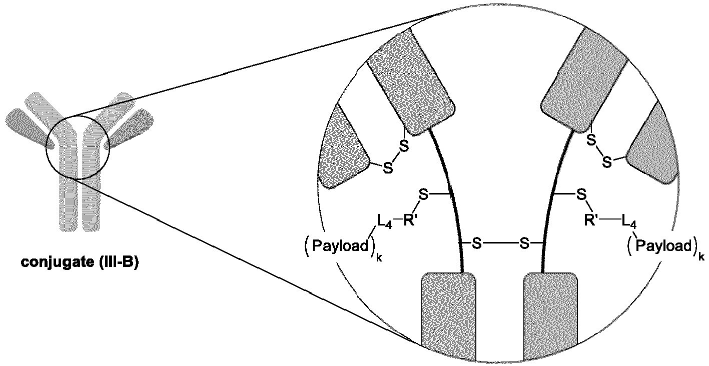
S
S
S
L4—R'
(Payload)k
S—S
S
S
S
R'—L4
(Payload)k
conjugate (III-B)

To a solution of Protein (100 µL, 40 µM in PBS pH 7.4) were added TCEP·HCl (10 mM in water, 2.4 µL, 6 equiv.). The mixture was incubated at 37°C for 2 hours, followed by cooling to 20°C. Subsequently, a solution of Zn(OAc)₂ (4 mM in water, 1.4 µL, 1.4 equiv.) was added to the mixture, followed by the addition of dehydroascorbic acid (DHAA, 10 mM in DMSO, 6 µL, 15 equiv.). The resulting mixture was incubated at 20°C for 1 hour, then purified using Bio-Spin^{®} P-30Gel Column (Bio-Rad) equilibrated with PBS (1X, pH 7.4) and cooled to 4°C. To the resulting mixture was added the solution of Payload (10 mM in DMSO, 4.0 µL, 10 equiv.) followed by EDTA (0.5 M in water, 0.5 µL, pH 8.0). The mixture was incubated until completion of the conjugation reaction (e.g. 1.5 hours at 4°C for maleimide-based payloads, 16h at 20°C for APN-based payloads). The resulting conjugate (III-B) was purified using Bio-Spin^{®} P-30Gel Column (Bio-Rad) equilibrated with PBS (1X, pH 7.4).

### General procedure E for hydrophobic interaction chromatography (HIC)

HIC was performed using a liquid chromatography system (Alliance HPLC, Waters, Manchester, UK) coupled to a 2487 UV/visible detector (Waters, Manchester, U.K.). Analyses were conducted on a TSKButyl-NPR, 3.5 × 4.6 mm column (Tosoh Bioscience, Tokyo, Japan) set at 20°C. The mobile phase A consisted of 1.5 M ammonium sulfate and 25 mM potassium phosphate at pH 7.0, while mobile phase B was a mixture of 25 mM potassium phosphate and 25% isopropanol at pH 7. Separation was achieved with a linear gradient of 0%-100% B over 12 min at a flow rate of 0.8 mL/min. Analyses were performed by injecting 20 µg of sample diluted to 1 mg/mL with mobile phase A and integrating the UV area at 210 nm for each species.

General procedure F for reverse-phase high performance liquid chromatography - mass spectrometry (RP-HPLC MS) characterization of the conjugates
RP-HPLC MS analyses were conducted using a ThermoFisher Ultimate 3000 HPLC system (Thermo Fisher Scientific, Germering, Germany), coupled to an Exactive^{™} Plus Extended Mass Range (EMR, Orbitrap analyzer, Thermo Fisher Scientific, Bremen, Germany). This setup was equipped with an HESI-II probe source operating in positive ion mode. Instrument control and data acquisition were handled using the Thermo Xcalibur^{™} mass spectrometry data system. For the analysis, an Agilent PLRP-S, 1000 Å, 2.1 × 50 mm, 5 µm column (P/N: PL1912-1502) was utilized and maintained at 40°C. Mobile phase A was 0.1% formic acid in DI water, mobile phase B was 0.1% formic acid in acetonitrile. The separation process involved a flow rate of 0.600 mL/min and employed the following gradient: 0-4 min, isocratic 20% B; 4-5 min, 20% to 40% B; 5-10 min, 40% to 70% B; 10-11 min, 70% to 90% B. Full MS spectra were acquired in positive polarity with a scan range of 800-4,000 m/z. The resolution was set at 70,000, with an AGC target of 1 × 106 ions. Sheath gas was set to 35 AU and auxiliary gas was set to 10 AU. The spray voltage was 2.2 kV, the capillary temperature was 250°C. Prior to analysis, all protein samples were deglycosylated using Endo S enzyme (New England Biolabs, P/N: P0741S) following manufacturer's instructions. For each analysis, 1 µg of the protein conjugate was injected. All MS data of the conjugates were deconvoluted and analyzed using UniDec software (version 5.2.1, Marty et al. Anal. Chem. 2015. DOI: 10.1021/acs.analchem.5b00140).

### General procedure G for peptide mapping

50 µg of ADC were solubilized 80 µL of 50 mM NH₄HCO₃, 0.1% RapiGest (Waters) at pH 7.8. IdeS digestion was performed by adding 50 units of FabRICATOR (Genovis) at 37°C for 30 min. Disulfide reduction was performed by incubating with 5 mM TCEP for 30 min at 57°C. Alkylation of cysteins was performed in 10 mM IAM in the dark at 20°C for 40 min.

Trypsin (Promega, V5111) was prepared by suspending 20 µg in 100 µL of H₂O. Digestion was performed by adding 5 µL of trypsin solution, which corresponds to a 1:50 enzyme:substrate ratio. Samples were incubated overnight at 20°C. The reaction was stopped by adding 1 µL of TFA. RapiGest was eliminated by incubation at 37°C for 30 min and centrifugation at 13,000 g for 5 min.

NanoLC-MS/MS analysis was performed using a nanoACQUITY Ultra-Performance-LC (Waters Corporation, Milford, USA) coupled to a Q-Exactive Plus mass spectrometer (Thermo Fisher Scientific, Bremen, Germany). The nanoLC system was composed of ACQUITY UPLC^{®} CSH130 C18 column (250 mm × 75 µm with a 1.7 µm particle size, Waters Corporation, Milford, USA) and a Symmetry C18 precolumn (20 mm × 180 µm with a 5 µm particle size, Waters Corporation, Milford, USA). The solvent system consisted of 0.1% formic acid in water (solvent A) and 0.1% formic acid in acetonitrile (solvent B). Sample was loaded into the enrichment column during 3 min at 5 µL/min with 99% of solvent A and 1% of solvent B. Elution of the peptides was performed at a flow rate of 350 nL/min with a 2-44% linear gradient of solvent B in 38 minutes, followed by a 44-64% linear gradient in 30 minutes.

The Q-Exactive Plus was operated in data-dependent acquisition mode by automatically switching between full MS and consecutive MS/MS acquisitions. Full-scan MS spectra were collected from 300 - 1,800 m/z at a resolution of 70,000 at 200 m/z with an automatic gain control target fixed at 3 × 106 ions and a maximum injection time of 50 ms. The top 10 precursor ions with an intensity exceeding 5 × 104 ions and charge states ≥ 2 were selected from each MS spectrum for fragmentation by higher-energy collisional dissociation. Spectra were collected at a resolution of 17,500 at 200 m/z with a fixed first mass of 100 m/z, an automatic gain control target fixed at 1 × 105 ions and a maximum injection time of 100 ms. Dynamic exclusion time was set to 3 s.

Peptides containing drug linker were identified by using Byos^{®} Desktop Software (Protein Metrics) or searched manually in raw data.

### General procedure H for middle analysis

50 µg of ADC were solubilized in 50 µL of 20 mM Tris-HCI at pH 7.5. IdeS digestion was performed by adding 50 units of FabRICATOR (Genovis) at 37°C for 30 min. In order to perform a strong denaturation, 25 mg of guanidine HCI was directly added, followed by reduction with 60 mM TCEP. After 60 min incubation at 57 °C, the reaction was quenched by adding 1% TFA.

rpLC-MS analyses on large antibody fragments were also performed using the BioAccord system. A volume equivalent to 3 µg of sample preparation, was injected on a BioResolve RP mAb Polyphenyl Column, 450Å, 2.7 µm, 2.1 mm × 50 mm (Waters, Manchester, UK, ref. 186008944) set at 80°C.

Ultrapure water for mobile phase A (0.1% FA) and acetonitrile for mobile phase B (0.1% FA) were used to perform the chromatographic separation. The gradient was generated at a flow rate of 300 µL/min and the elution program started at 20% mobile phase B from initial conditions to reach 40% in 10 minutes. The temperature column was maintained at 80 °C. The ACQUITY RDa was operated in positive mode with a capillary voltage of 1.5 kV while sample cone and desolvation temperature were set to 50 V and 550 °C, respectively. Acquisitions were performed under full scan mode and high mass range (400-7,000 m/z) with 2 Hz scan rate. The mass spectrometer was calibrated using the ACQUITY RDa Detector Calibrant and Wash Kit (Waters, Manchester, UK, ref. 186009013). The ACQUITY RDa Waters connect LockMass Kit (Waters, Manchester, UK, ref. 186009298) was used before and after each injection in order to compensate for mass variations due to temperature changes in the laboratory. UNIFI Scientific Information System (Waters, Manchester, UK) was used as a single solution to encompass data acquisition and processing. 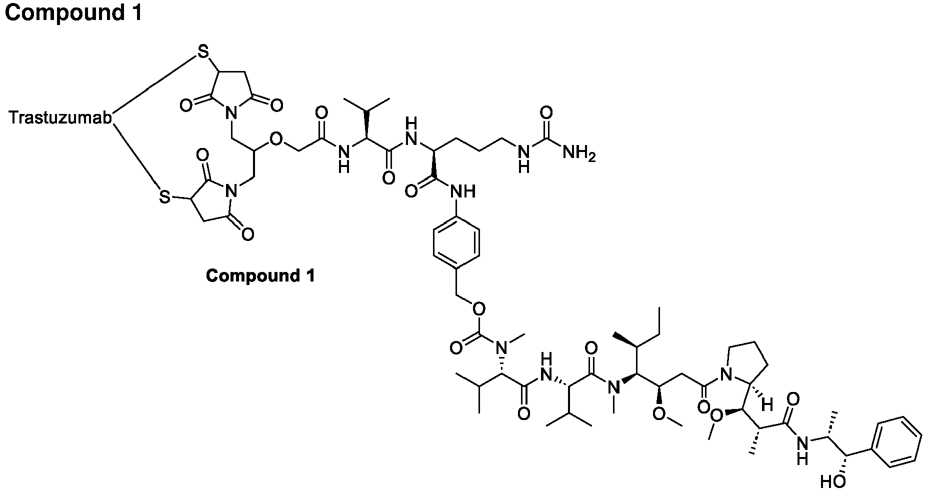 Compound 1 was prepared following General procedure C, using Trastuzumab as protein and Bi-MC-VC-MMAE (CAS 1620837-70-8) as thiol-reactive payload. The product was characterized by RP-HPLC MS following General procedure F. RP-HPLC MS results: Expected mass 147 279 Da; observed mass 147 295 Da. 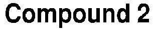 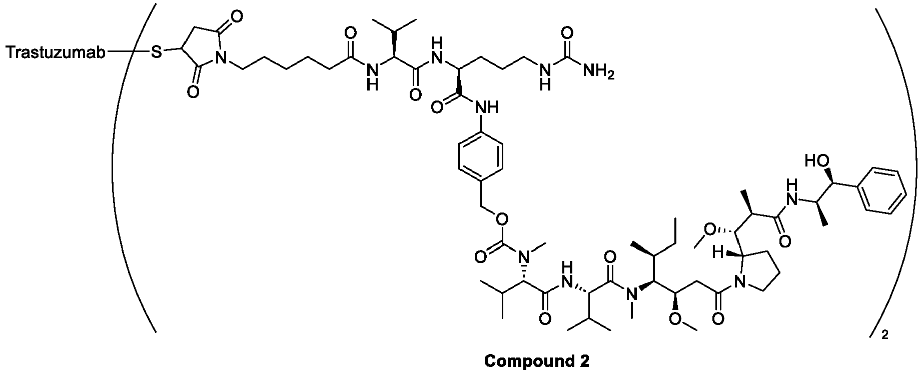 Compound 2 was prepared following General procedure D, using Trastuzumab as protein and MC-VC-MMAE (CAS 646502-53-6) as thiol-reactive payload. The product was characterized by RP-HPLC MS following General procedure F. RP-HPLC MS results: Expected mass 148 497 Da; observed mass 148 509 Da.

Compound 2 was characterized by peptide mapping following General Procedure G and by middle analysis following General Procedure H. Both analyses confirmed the predominant conjugation site for the payload at C229 on the heavy chain of trastuzumab, as numbered in IMGT/2Dstructure-DB (INN number 7637).

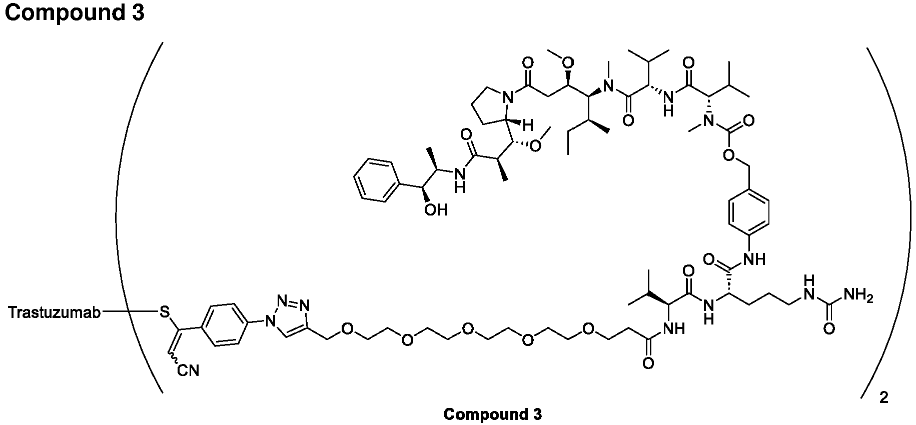

Compound 3 was prepared following General procedure D, using Trastuzumab as protein and APN-VC-MMAE (CAS 2252392-31-5) as thiol-reactive payload. The product was characterized by RP-HPLC MS following General procedure F. RP-HPLC MS results: Expected mass 149 019 Da; observed mass 149 028 Da.

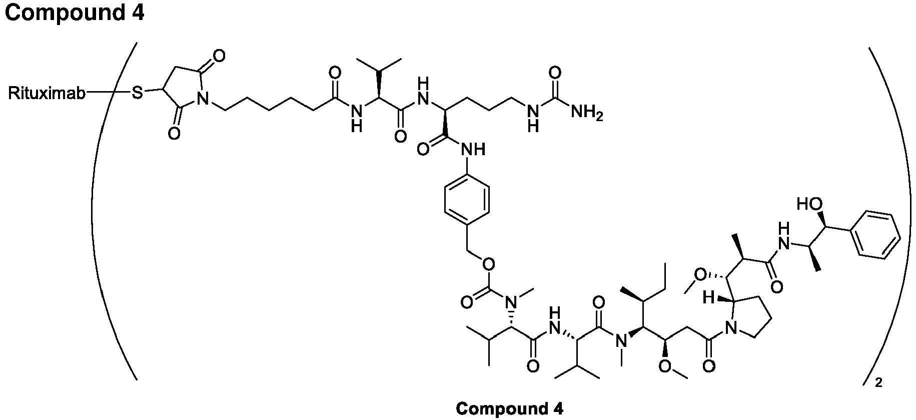

Compound 4 was prepared following General procedure D, using Rituximab as protein and MC-VC-MMAE (CAS 646502-53-6) as thiol-reactive payload. The product was characterized by RP-HPLC MS following General procedure F. RP-HPLC MS results: Expected mass 147 529 Da; observed mass 147 524 Da.

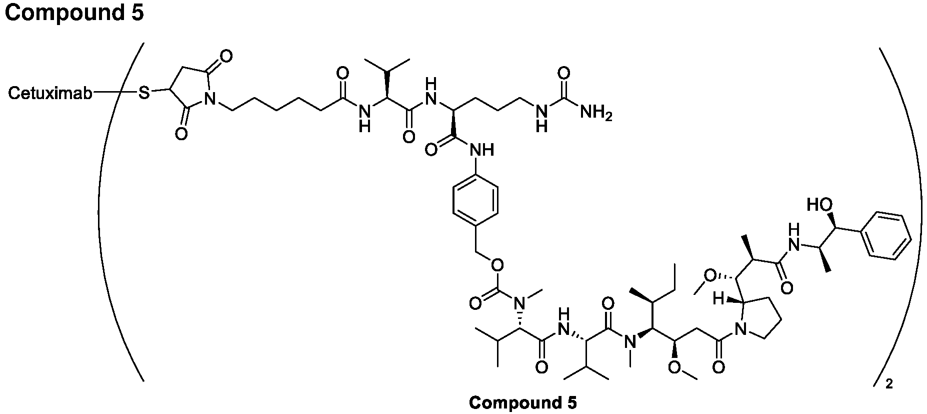

Compound 5 was prepared following General procedure D, using Cetuximab as protein and MC-VC-MMAE (CAS 646502-53-6) as thiol-reactive payload. The product was characterized by RP-HPLC MS following General procedure F. RP-HPLC MS results: Expected mass 152 937 Da; observed mass 152 936 Da. 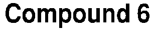 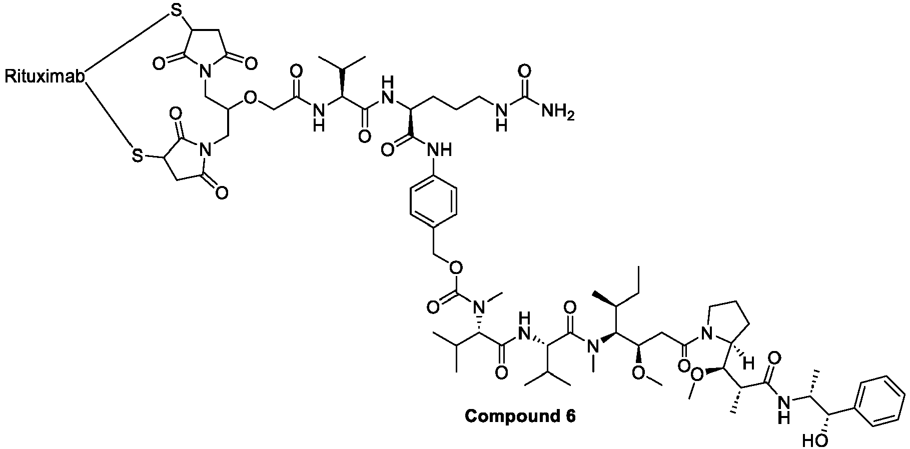 Compound 6 was prepared following General procedure C, using Rituximab as protein and Bi-MC-VC-MMAE (CAS 1620837-70-8) as thiol-reactive payload. The product was characterized by RP-HPLC MS following General procedure F. RP-HPLC MS results: Expected mass 146 311 Da; observed mass 146 315 Da. 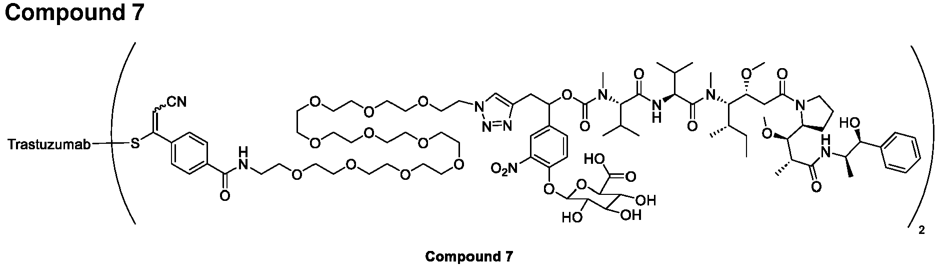 Compound 7 was prepared following General procedure D, using Trastuzumab as protein and APN-Glu-MMAE (CAS 2252392-28-0) as thiol-reactive payload. The product was characterized by RP-HPLC MS following General procedure F. RP-HPLC MS results: Expected mass 149 477 Da; observed mass 149 477 Da.

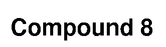

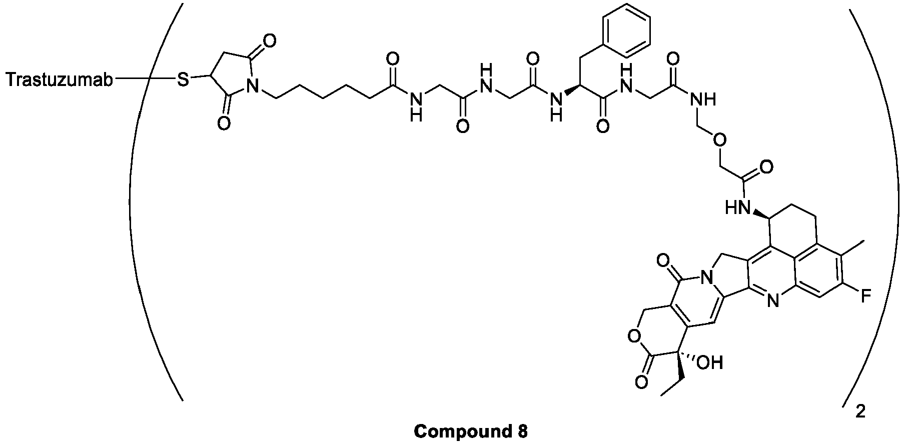

Compound 8 was prepared following General procedure D, using Trastuzumab as protein and Deruxtecan (CAS 1599440-13-7) as thiol-reactive payload. The product was characterized by RP-HPLC MS following General procedure F. RP-HPLC MS results: Expected mass 147 931 Da; observed mass 147 946 Da. 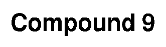 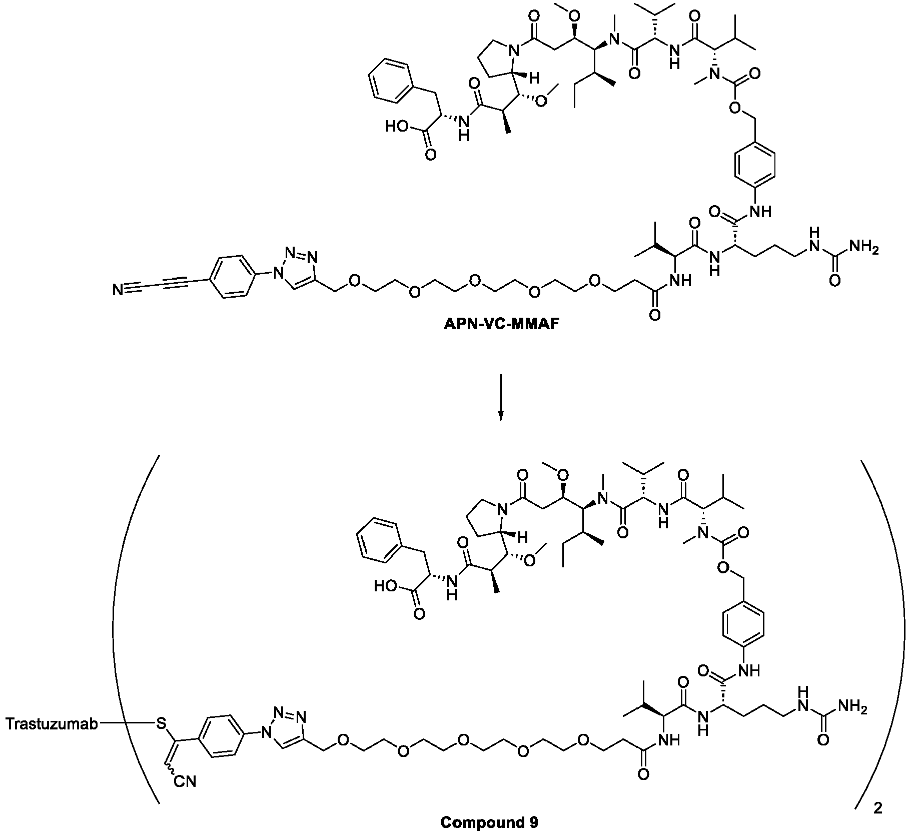 Compound 9 was prepared following General procedure D, using Trastuzumab as protein and APN-VC-MMAF as thiol-reactive payload. The product was characterized by RP-HPLC MS following General procedure F. RP-HPLC MS results:
Expected mass 149 049 Da; observed mass 149 057 Da. 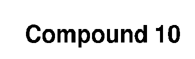 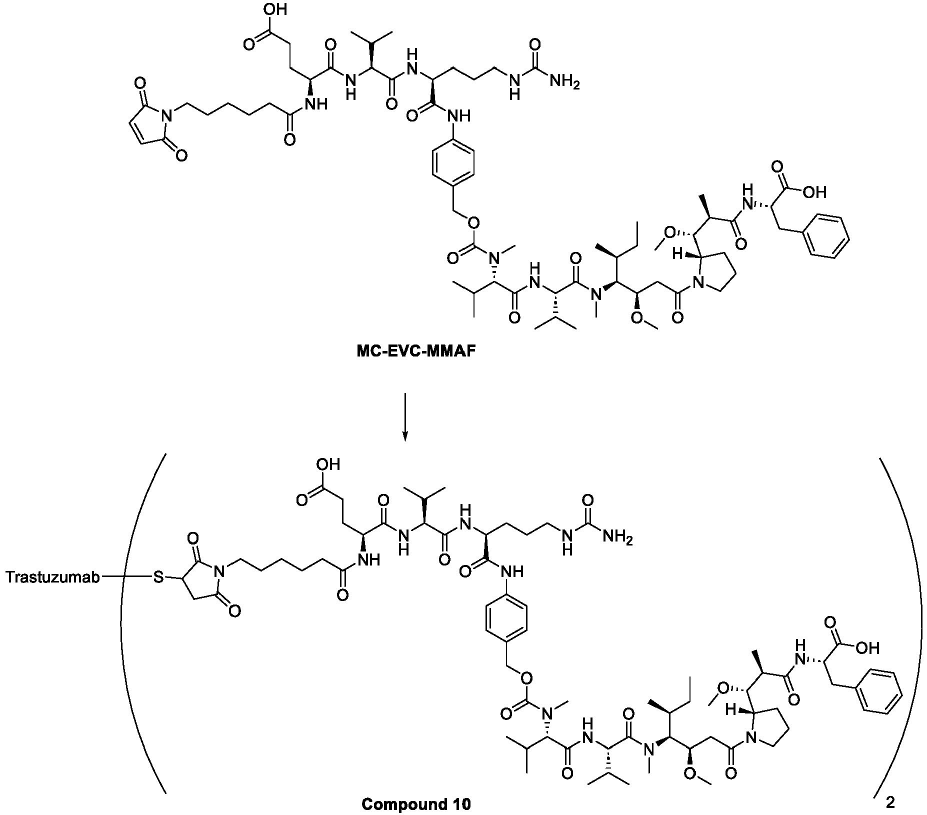 Compound 10 was prepared following General procedure D, using Trastuzumab as protein and MC-EVC-MMAF as thiol-reactive payload. The product was characterized by RP-HPLC MS following General procedure F. RP-HPLC MS results:
Expected mass 148 783 Da; observed mass 148 794 Da. 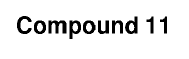 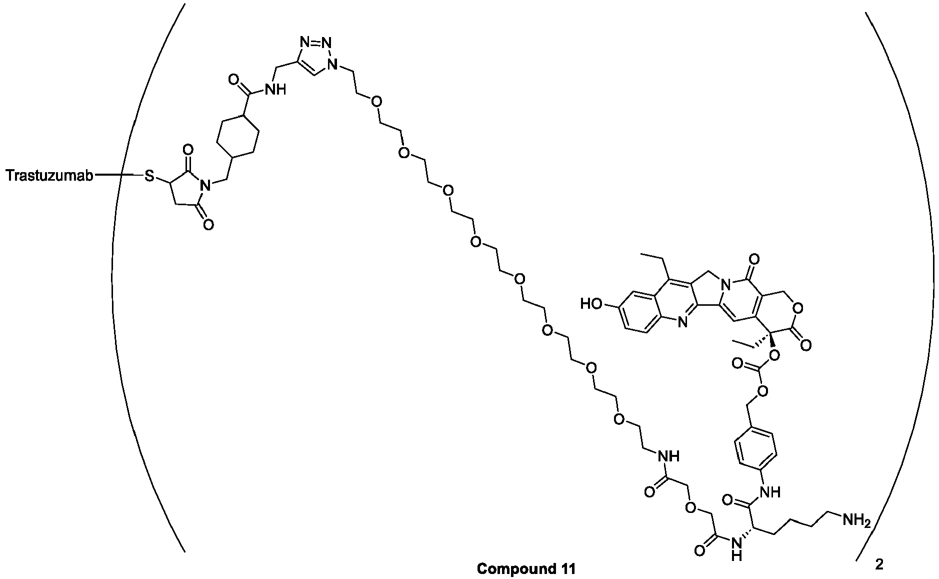 Compound 11 was prepared following General procedure D, using Trastuzumab as protein and CL2A-SN-38 (CAS 1279680-68-0) as thiol-reactive payload. The product was characterized by RP-HPLC MS following General procedure F. RP-HPLC MS results: Expected mass 148 825 Da; observed mass 148 838 Da.

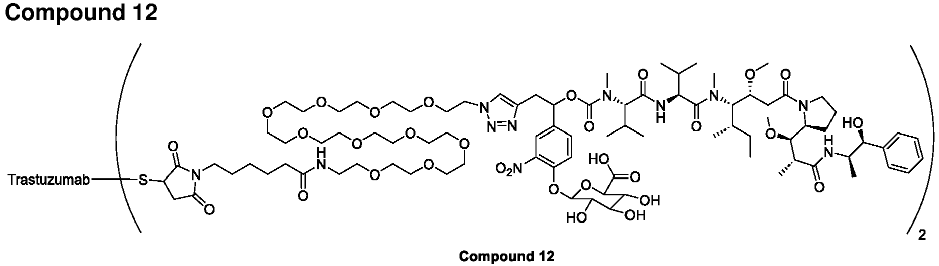

Compound 12 was prepared following General procedure D, using Trastuzumab as protein and MC-Glu-MMAE (CAS 1803182-85-5) as thiol-reactive payload. The product was characterized by RP-HPLC MS following General procedure F. RP-HPLC MS results: Expected mass 149 557 Da; observed mass 149 572 Da. 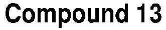 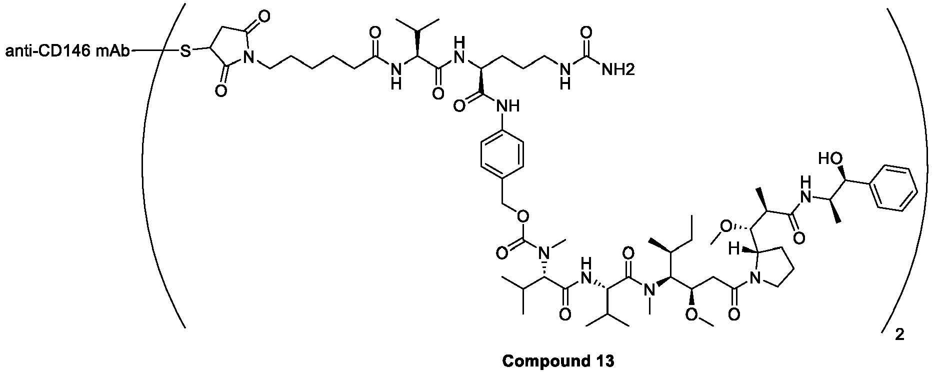 Compound 13 was prepared following General procedure D, using anti-CD146 mAb (described in WO2019068842A1) as protein and MC-VC-MMAE (CAS 646502-53-6) as thiol-reactive payload. The product was characterized by RP-HPLC MS following General procedure F. RP-HPLC MS results: Expected mass 148 185 Da; observed mass 148 182 Da.

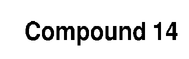

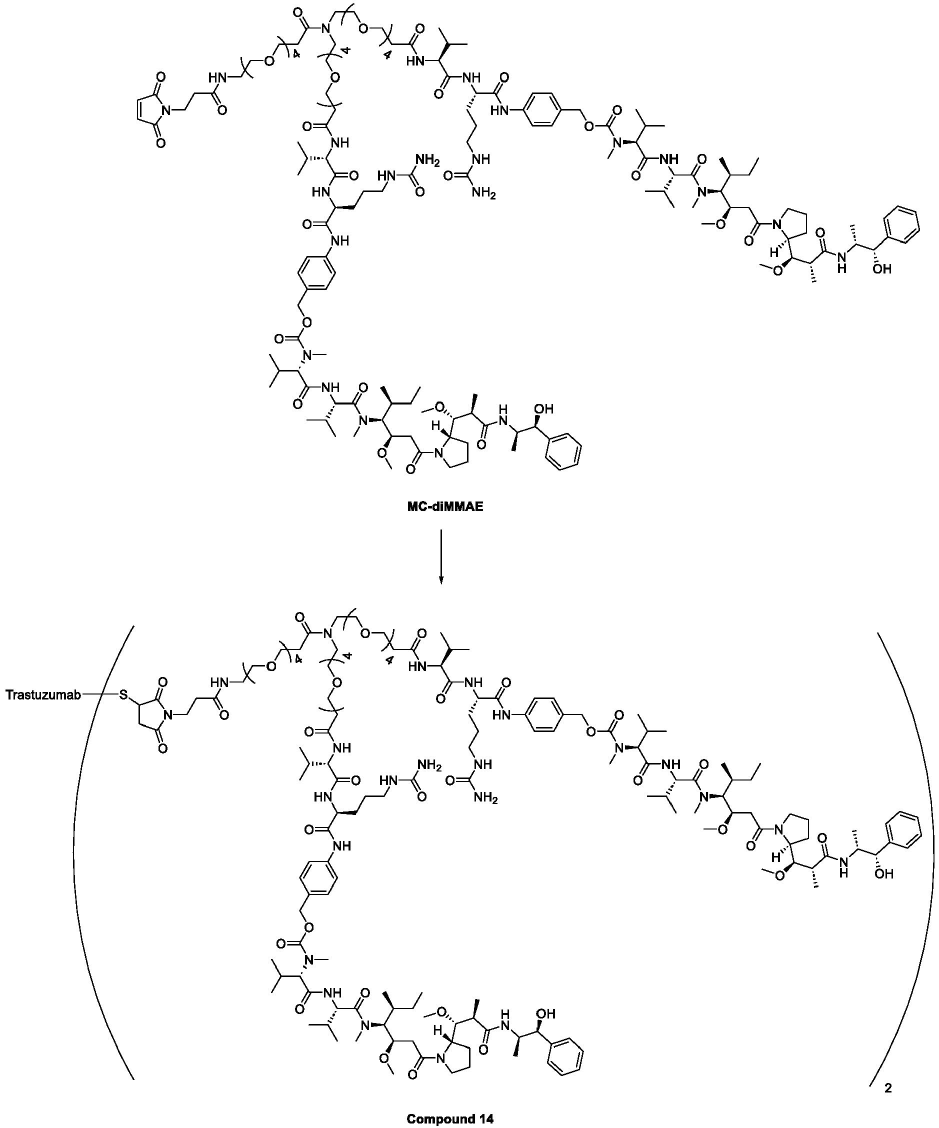

Compound 14 was prepared following General procedure D, using Trastuzumab as protein and MC-diMMAE as thiol-reactive payload. The product was characterized by RP-HPLC MS following General procedure F. RP-HPLC MS results:
Expected mass 152 111 Da; observed mass 152 123 Da.

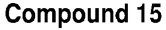

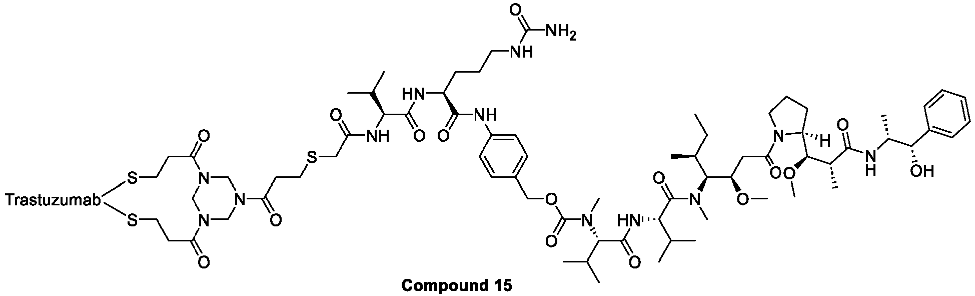

Compound 15 was prepared following General procedure C, using Trastuzumab as protein and Py-MAA-Val-Cit-PAB-MMAE (CAS 2247398-68-9) as thiol-reactive payload. The product was characterized by RP-HPLC MS following General procedure F. RP-HPLC MS results:
Expected mass 147 312 Da; observed mass 147 319 Da. 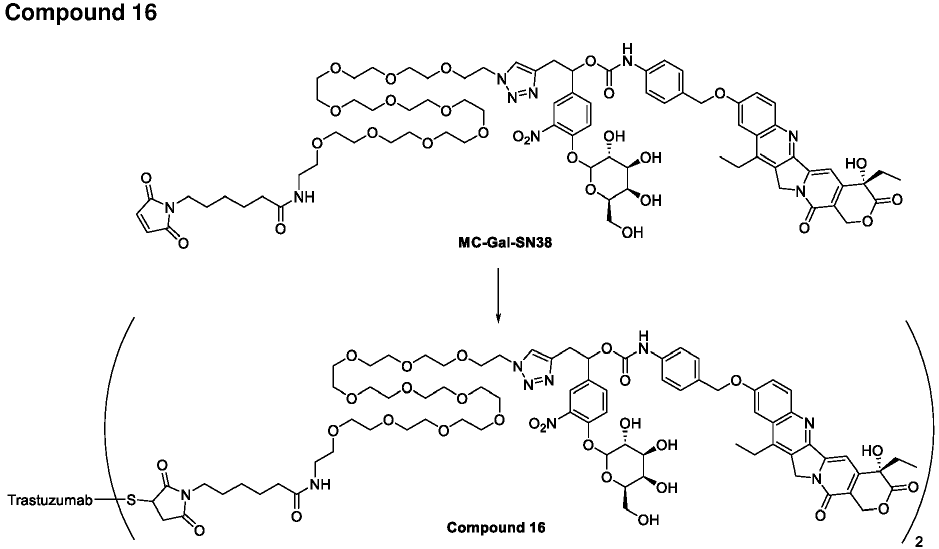 Compound 16 was prepared following General procedure D, using Trastuzumab as protein and MC-Gal-SN38 as thiol-reactive payload. The product was characterized by RP-HPLC MS following General procedure F. RP-HPLC MS results:
Expected mass 149 135 Da; observed mass 149 144 Da. 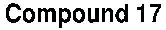 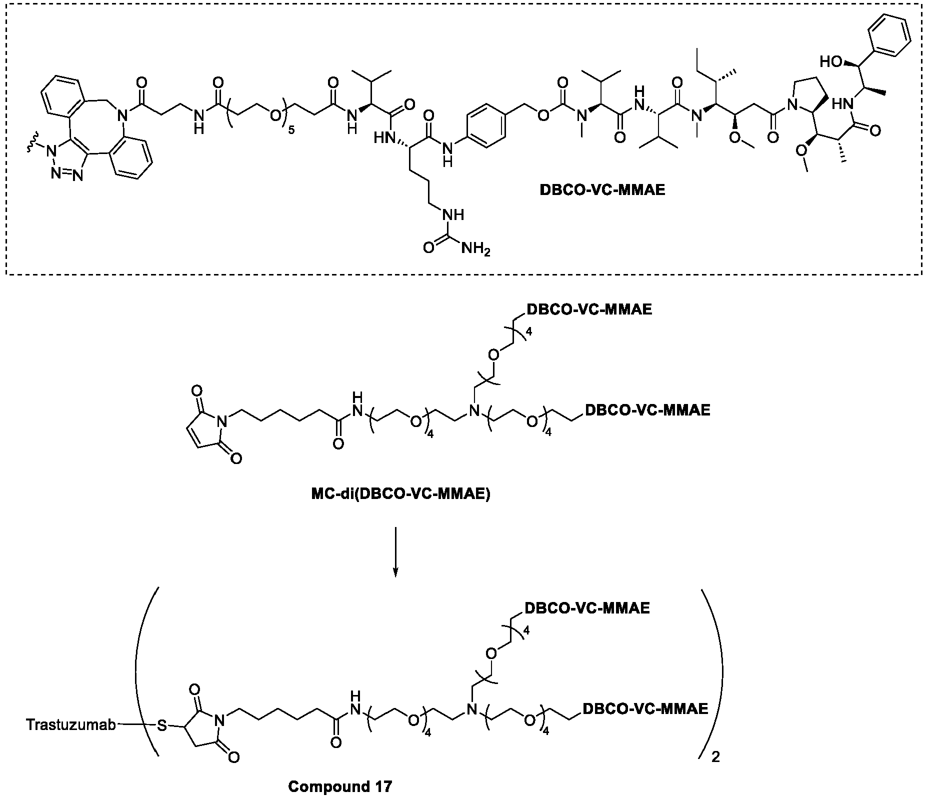 Compound 17 was prepared following General procedure D, using Trastuzumab as protein and MC-di(DBCO-VC-MMAE) as thiol-reactive payload. The product was characterized by RP-HPLC MS following General procedure F. RP-HPLC MS results:
Expected mass 154 511 Da; observed mass 154 521 Da. 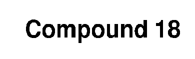 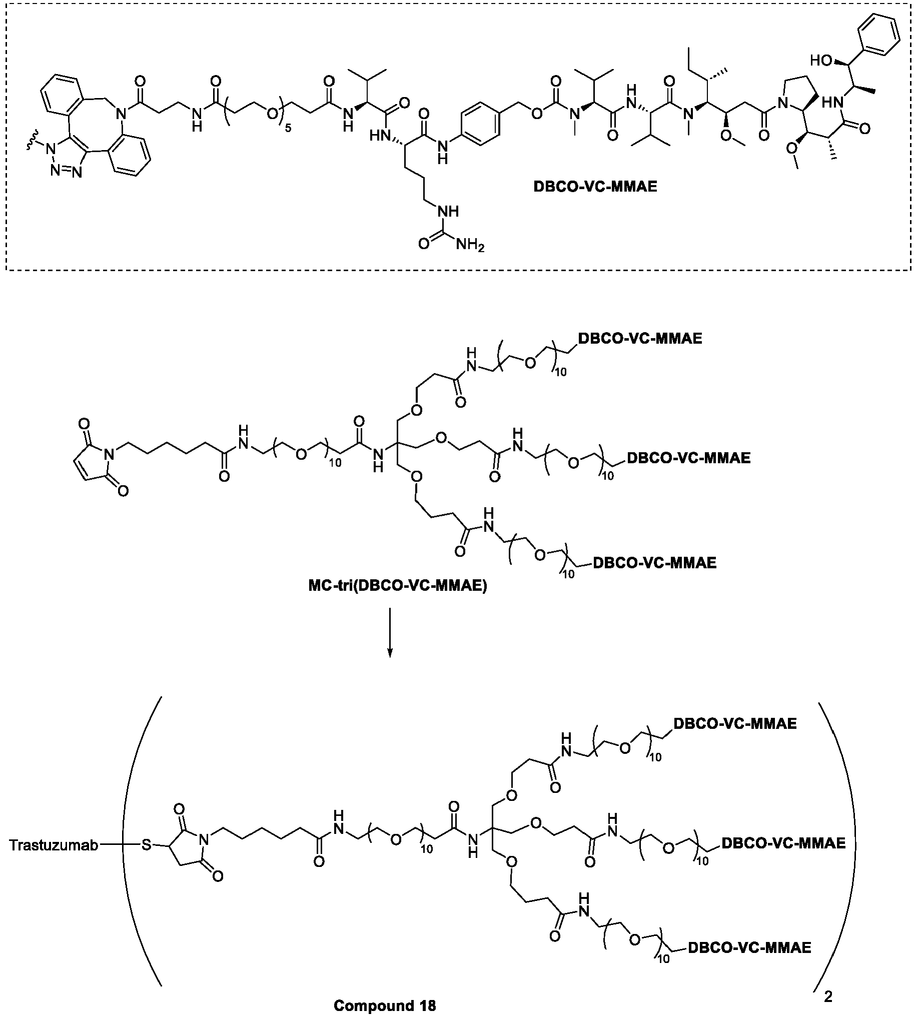 Compound 18 was prepared following General procedure D, using Trastuzumab as protein and MC-tri(DBCO-VC-MMAE) as thiol-reactive payload. The product was characterized by RP-HPLC MS following General procedure F. RP-HPLC MS results:
Expected mass 161 241 Da; observed mass 161 221 Da. 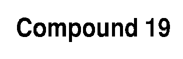 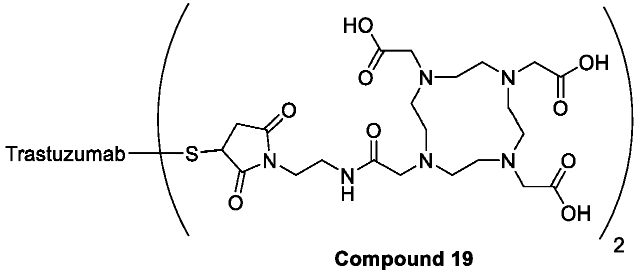 Compound 19 was prepared following General procedure D, using Trastuzumab as protein and Maleimide-DOTA (CAS 1006711-90-5) as thiol-reactive payload. The product was characterized by RP-HPLC MS following General procedure F. RP-HPLC MS results: Expected mass 146 917 Da; observed mass 146 920 Da. 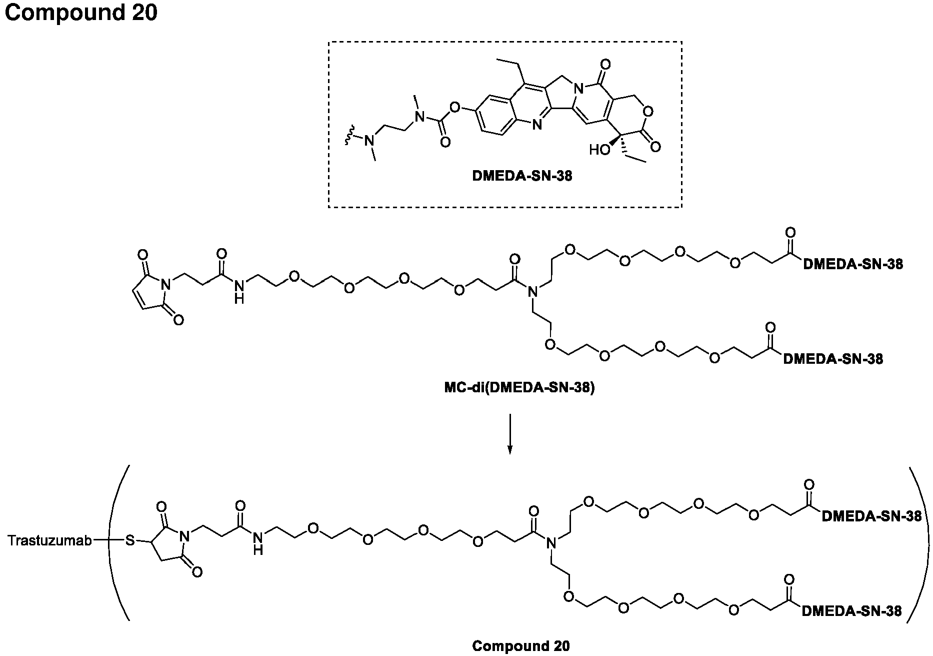 Compound 20 was prepared following General procedure D, using Trastuzumab as protein and MC-di(DMEDA-SN-38) as thiol-reactive payload. The product was characterized by RP-HPLC MS following General procedure F. RP-HPLC MS results:
Expected mass 149 643 Da; observed mass 149 651 Da. 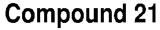 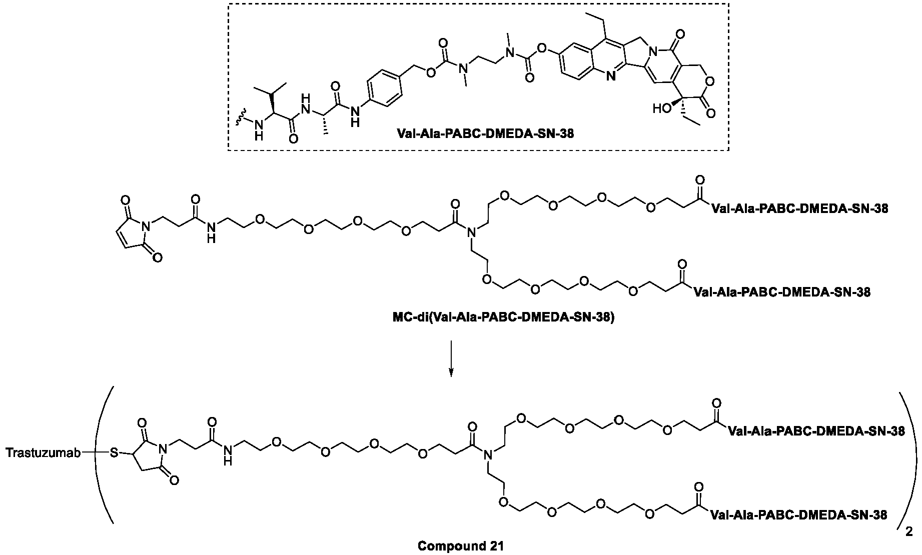 Compound 21 was prepared following General procedure D, using Trastuzumab as protein and MC-di(Val-Ala-PABC-DMEDA-SN-38) as thiol-reactive payload. The product was characterized by RP-HPLC MS following General procedure F. RP-HPLC MS results: Expected mass 150 919 Da; observed mass 150 932 Da. 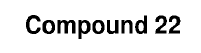 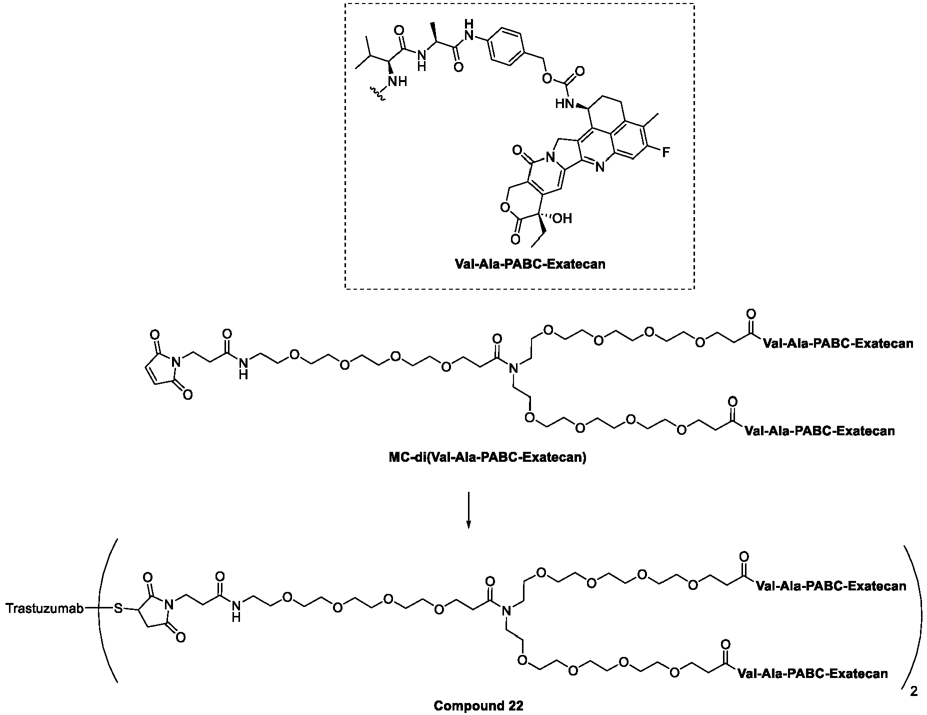 Compound 22 was prepared following General procedure D, using Trastuzumab as protein and MC-di(Val-Ala-PABC-Exatecan) as thiol-reactive payload. The product was characterized by RP-HPLC MS following General procedure F. RP-HPLC MS results: Expected mass 150 635 Da; observed mass 150 642 Da. 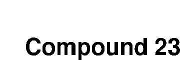 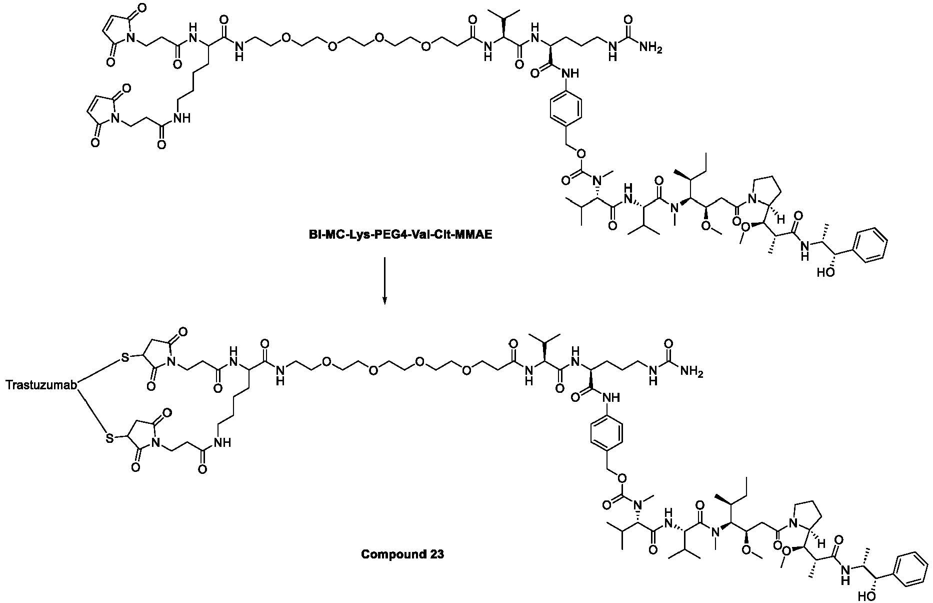 Compound 23 was prepared following General procedure C, using Trastuzumab as protein and Bi-MC-Lys-PEG4-Val-Cit-MMAE as thiol-reactive payload. The product was characterized by RP-HPLC MS following General procedure F. RP-HPLC MS results: Expected mass 147 665; observed mass 147 669 Da. 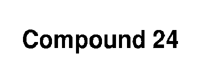 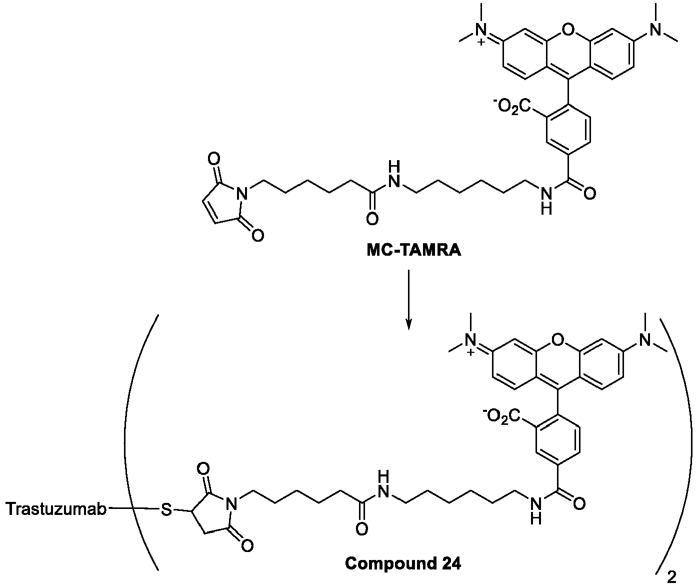 Compound 24 was prepared following General procedure D, using Trastuzumab as protein and MC-TAMRA as thiol-reactive payload. The product was characterized by RP-HPLC MS following General procedure F. RP-HPLC MS results:
Expected mass 147 307 Da; observed mass 147 312 Da. 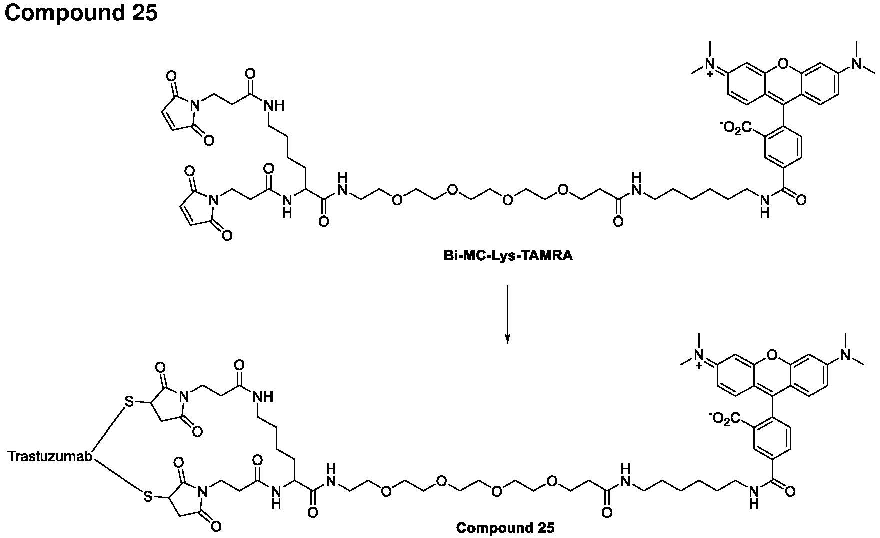 Compound 25 was prepared following General procedure C, using Trastuzumab as protein and Bi-MC-Lys-TAMRA as thiol-reactive payload. The product was characterized by RP-HPLC MS following General procedure F. RP-HPLC MS results:
Expected mass 147 070 Da; observed mass 147 074 Da. 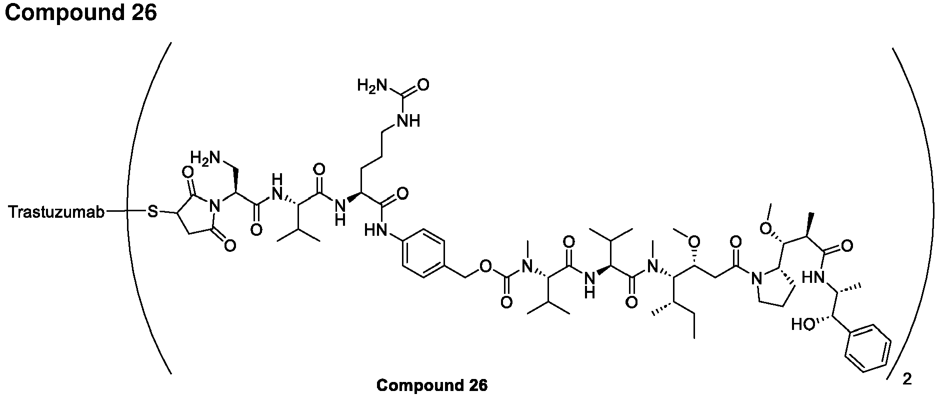 Compound 26 was prepared following General procedure C, using Trastuzumab as protein and mDPR-Val-Cit-PAB-MMAE (CAS 2185872-76-6) as thiol-reactive payload. The product was characterized by RP-HPLC MS following General procedure F. RP-HPLC MS results: Expected mass 148 443 Da; observed mass 148 451 Da.

## Claims

1. A coordination complex of protein with a divalent metal atom M, represented by formula (II):
[Ab'-M] (II)
Wherein
Ab' is a protein or fragment thereof comprising two heavy chains and two light chains, each chain comprising one or more cysteine residue involved in inter chains disulfide bridges,
wherein each heavy chain comprises one N-proximal inter(heavy chains) cysteine residue that is located nearest to the N-terminal end of said heavy chain,
And wherein the Sulfur atom of each said N-proximal inter(heavy chains) cysteine residue is reduced, and the remaining interchain cysteine residues form interchain disulfide bridges;
M is a divalent transition metal;
Wherein M bridges together the two reduced Sulfur atoms, so as to form the following group:
-S-M-S- within the coordination complex;
And the salts thereof;
**Characterized in that** the complexion of M is site specific.

2. The complex of formula (II) according to claim 1 wherein in formula (II) M is Zn.

3. The complex of formula (II) according to claim 1 or 2 wherein in formula (II) Ab' is an antibody, in particular Immunoglobulin G, or a derivative thereof.

4. Process of preparation of the complex of formula (II), said process comprising:
Reacting a protein complex of formula (I):
[Ab'_{Red}-M] (I)
With an oxidizing agent;
wherein
Ab'_{Red} is a protein or fragment thereof comprising two heavy chains and two light chains,
each chain comprising one or more cysteine residue involved in inter chains disulfide bridges,
wherein each heavy chain comprises one N-proximal inter(heavy chains) cysteine residue that is located nearest to the N-terminal end of said heavy chain,
And wherein each Sulfur atom of each said N-proximal inter(heavy chains) cysteine residue is reduced and the two reduced Sulfur atoms form together with the M atom the following group: -S-M-S- within the coordination complex;
and the Sulfur atoms of the remaining interchain cysteine residues are reduced in the form of thiol -SH groups;
M is defined as in claim 1 or 2.

5. The process according to claim 4 wherein said oxidizing agent is dehydroascorbic acid (DHAA).

6. A coordination complex of protein of formula (I):
[Ab'_{Red}-M] (I)
Where Ab'_{Red} and M are defined as in claim 4.

7. The process of preparation of the complex of formula (I) according to claim 6, said process comprising:
- reacting Ab with a reducing agent so as to form Ab_{Red}, followed by
- reacting Ab_{Red} with 0.5 to 2.5, preferably between 1 and 2 equivalents of a salt of metal M so as to form the Metal coordination complexes [Ab'_{Red}-M];
Wherein
Ab is a protein or fragment thereof comprising two heavy chains and two light chains, each chain comprising one or more cysteine residue involved in inter chains disulfide bridges,
Ab_{Red} refers to reduced Ab wherein all Sulfur atoms of said interchain cysteine residues are reduced and in the form of thiol -SH groups.

8. A protein conjugate of formula (III):
(T)ₚ-Ab'-(L-(Payload)ₘ)ₙ (III)
Having
- a controlled drug-to-antibody ratio (DAR) of (m x n); and
- a site specific conjugation at one or two of the N-proximal inter(heavy chains) cysteine residue that is located nearest to the N-terminal end of said heavy chain;
Where in formula (III):
Ab' is a protein or fragment thereof comprising two heavy chains and two light chains, each chain comprising one or more cysteine residue involved in inter chains disulfide bridges,
wherein each heavy chain comprises one N-proximal inter(heavy chains) cysteine residue that is located nearest to the N-terminal end of said heavy chain,
And wherein each Sulfur atom of each said N-proximal inter(heavy chains) cysteine residue is reduced and covalently bound to either -(L-(Payload)ₘ or optional T fragment, and the remaining interchain cysteine residues form interchain disulfide bridges;
n is 1 or 2;
m is 1, 2, 3, 4, 5 or 6;
p is 0 or 1;
T, if present, is a terminal group covalently bound to one of said two reduced Sulfur atoms of said N-proximal inter(heavy chains) cysteine residues of Ab' that is not covalently bound to L;
each L, identical or different, is a linear or branched di-, tri-, tetra-, penta-, hexa- or hepta-valent linker moiety covalently binding Payload to said Ab', where said L is attached to Ab' through one or two of said reduced Sulfur atoms of said N-proximal inter(heavy chains) cysteine residue and binds said Ab' with one or more Payload;
Payload is a molecule exerting a biological activity selected from the group consisting in diagnostic agents, therapeutic agents and labelling agents.

9. The protein conjugate of formula (III) according to claim 8 wherein the conjugate of formula (III) is of formula (III-A):
Ab'-L-(Payload) (III-A)
Having a controlled drug-to-antibody ratio (DAR) of 1;
Where in formula (III-A):
Ab' and Payload are defined as in claim 8;
L is a trivalent linker moiety covalently binding together the two said reduced Sulfur atoms of Ab' with Payload.

10. The protein conjugate of formula (III) according to claim 9 wherein the conjugate of formula (III-A) comprises the following fragment: 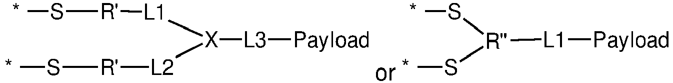
Wherein each S designates the reduced Sulfur atom of each said N-proximal inter(heavy chains) cysteine residue;
* designates the attachment of said Sulfur atom to the rest of Ab' at each heavy chain through the corresponding N-proximal inter(heavy chains) cysteine residue;
L₁, L₂ and L₃ identical or different are optional divalent Spacer moieties;
X is chosen from linear, branched or cyclic trivalent moieties, comprising 1 to 9 nitrogen atoms and from 0 to 30 carbon atoms;
R' is a group formed as a result of a reaction between a thiol and a thiol-reactive group, such as maleimide, 3-arylpropiolonitrile, bromo-maleimide, chloroacetamide, bromoacetamide, iodoacetamide, acrylamide, vinyl sulfone, pyridyl disulfide, alpha-halo carbonyl, vinyl pyridine;
R" is a group formed as a result of a reaction between two thiols and a bifunctional thiol-reactive group, such as 3,4-disubstituded maleimide, 3,3'-(phenylene)dipropiolonitrile, dibromopyridazinedione, bissulfone, allyl sulfone, 3-bromomaleimide or arsenous acid.

11. The protein conjugate of formula (III) according to claim 8 wherein the conjugate of formula (III) is of formula (III-B):
Ab'-(L-(Payload)ₘ)₂ (III-B)
Having a controlled drug-to-antibody ratio (DAR) of 2m;
Where in formula (III-B):
m, Ab' and Payload are defined as in claim 8;
Each of the two L is di-, tri-, tetra- or penta-valent linear or branched linker moiety covalently binding one of said reduced Sulfur atoms of said N-proximal inter(heavy chains) cysteine residue of Ab' with m Payload(s).

12. The protein conjugate of formula (III) according to claim 11 wherein the conjugate of formula (III-B) comprises two following fragments:
* -S-R'-L4-(Payload)ₘ
Wherein
m and Payload are defined as in claim 8;
S designates the reduced Sulfur atom of each of the two said N-proximal inter(heavy chains) cysteine residues, respectively;
* designates the attachment of said Sulfur atom to the rest of Ab' at each heavy chain through the corresponding N-proximal inter(heavy chains) cysteine residue;
Each L₄ identical or different is an optional linear or branched Spacer moiety;
R' is a group formed as a result of a reaction between the reduced thiol group of Ab' and a thiol-reactive group, such as maleimide, 3-arylpropiolonitrile, bromo-maleimide, chloroacetamide, bromoacetamide, iodoacetamide, acrylamide, vinyl sulfone, pyridyl disulfide, alpha-halo carbonyl, vinyl pyridine.

13. The protein conjugate of formula (III) according to claim 8 wherein the conjugate of formula (III) is of formula (III-C):
T-Ab'-L-(Payload)ₘ (III-C)
Having a controlled drug-to-antibody ratio (DAR) of m;
Where in formula (III-C):
m, Ab' and Payload are defined as in claim 8;
L is di-, tri-, tetra- or penta-valent linear or branched linker moiety covalently binding one of said reduced Sulfur atoms of the two N-proximal inter(heavy chains) cysteine residues of Ab' with m Payload(s); and
T is defined as in claim 8 and is covalently bound to Ab' through the other one of said reduced Sulfur atoms of the two N-proximal inter(heavy chains) cysteine residues of Ab'.

14. The protein conjugate of formula (III) according to claim 13 wherein the conjugate of formula (III-C) comprises each of the two following fragments:
* -S-R'-L4-(Payload)ₘ 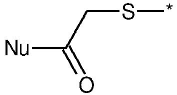
Wherein
Payload and m are defined as in claim 8;
Each S designates one reduced Sulfur atom of each of the two said N-proximal inter(heavy chains) cysteine residues;
Each * designates the attachment of said Sulfur atom to the rest of Ab' at each heavy chain through the corresponding N-proximal inter(heavy chains) cysteine residue;
Each L₄ identical or different is an optional linear or branched Spacer moiety;
R' is a group formed as a result of a reaction between the reduced thiol group of Ab' and a thiol-reactive group, such as maleimide, 3-arylpropiolonitrile, bromo-maleimide, chloroacetamide, bromoacetamide, iodoacetamide, acrylamide, vinyl sulfone, pyridyl disulfide, alpha-halo carbonyl, vinyl pyridine;
Nu is a nucleophilic moiety chosen from OH, -NH₂, -NH-NH₂, -NH-OH, -NH-R₁, -NR₁R₂, Where R₁, R₂ and R₃ are chosen from -CH₂-CONH₂, -CH₂-COOH, C1-C12 alkyl, C6-C12 aryl, C5-C12 heteroaryl; -(CH₂-CH₂-O-)ᵣ-CH₃ groups; in which r is an integer ranging from 1 to 24.

15. The process of preparation of a protein conjugate of formula (III) according to anyone of claims 8 to 14, said process comprising complexing said metal atom from said compound of formula (II) as defined in anyone of claims 1 to 3 and conjugating the obtained de-complexed compound with 1 to n conjugating fragments L-(Payload)ₘ, where L, Payload, m and n are defined as in claims 8 to 14.

16. A mixture comprising the protein conjugate of formula (III) as defined in anyone of claims 8 to 14, in at least 50% in number, preferably at least 60%, more preferably at least 70%, still more preferably at least 80%.

17. A pharmaceutical composition comprising an effective amount of the protein conjugate of formula (III) according to anyone of claims 8 to 14 or a mixture according to claim 16, together with a pharmaceutically acceptable carrier or vehicle.

18. The protein conjugate of formula (III) according to anyone of claims 8 to 14 or the mixture according to claim 16 for use for treating a condition or disorder chosen from the group consisting in tumor, cancer, autoimmune disease, or infectious disease in a subject.
